# EUROPEAN PATENT APPLICATION

(11) **EP 4 207 205 A1**
(43) Date of publication of application: **05.07.2023**
(21) Application number: 21861755.3
(22) Date of filing: 30.08.2021
(51) Int. Cl.: G16C 20/30, G06N 20/00

(54) **INFERENCE DEVICE, INFERENCE METHOD, INFERENCE PROGRAM, MODEL GENERATING METHOD, INFERENCE SERVICE PROVIDING SYSTEM, INFERENCE SERVICE PROVIDING METHOD, AND INFERENCE SERVICE PROVIDING PROGRAM**

(30) Priority: 31.08.2020 JP 2020146402
(71) Applicant: Takeda Pharmaceutical Company Limited, Osaka-shi, Osaka 541-0045 (JP)
(72) Inventor: MAEDA, Tomohiro, Fujisawa-shi, Kanagawa 251-0012 (JP); OGAWA, Nozomi, Fujisawa-shi, Kanagawa 251-0012 (JP)
(74) Representative: Carpmaels & Ransford LLP
(86) International application number: PCT/JP2021/031711
(87) International publication number: WO 2022/045334

(57) **Abstract**

An operation of designing or selecting chemical structure information on a lipid molecule forming a particle encapsulating an active ingredient is supported. An inference device includes an acquiring unit configured to acquire input data including at least chemical structure information on a lipid molecule, and a learned model generated by performing a learning process on a learning model that associates input data including at least chemical structure information on a lipid molecule with a transfection efficiency of an active ingredient encapsulated in a particle containing the lipid molecule into a cell and/or a cell survival rate. The learned model infers a transfection efficiency and/or a cell survival rate associated with the input data newly acquired by the acquiring unit.

## Description

### Technical Field

The present disclosure relates to an inference device, an inference method, an inference program, a model generating method, an inference service providing system, an inference service providing method, and an inference service providing program.

### Background of the Invention

A drug delivery system (DDS) using a particle containing lipid molecules for introducing an active ingredient such as a nucleic acid into a cell with high efficiency is known. In the system, by causing the particle containing lipid molecules to encapsulate an active ingredient, a complex particle is formed, and the active ingredient is introduced into a cell via the complex particle (transfection). Such a DDS is used not only for transfection into cells in a living organism by administration to a living organism but also for transfection into cells outside a living organism (in vitro, in situ, or ex vivo).

### Related Art Documents

### Patent Documents

Patent Document 1: International Publication Pamphlet No. WO 2016/021683
Patent Document 2: International Publication Pamphlet No. WO 2019/131839
Patent Document 3: International Publication Pamphlet No. WO 2020/032184

### Summary of the Invention

### Problems to be solved by the invention

With respect to the above, because the design and selection of the chemical structure of the lipid molecule forming the particle encapsulating the active ingredient are generally performed manually, the design and selection of an appropriate lipid molecule according to the purpose largely depend on experience and know-how of a skilled person thereof. Additionally, because an operation of evaluating a lipid molecule having a designed or selected chemical structure by an experiment and performing design or selection again in accordance with the evaluation result is repeatedly performed, it takes time to search for a chemical structure of a more appropriate lipid molecule. Furthermore, it is difficult to accumulate the know-how for designing or selecting chemical structures of lipid molecules suitable for various active ingredients and various purposes only by searching for lipid molecules suitable for limited types of active ingredients and purposes.

The present disclosure aims to support the operation of designing or selecting the chemical structure of the lipid molecule forming the particle encapsulating the active ingredient.

### Means for solving the problem

As a result of eagerly studying the above problem, the present inventors have found that a learning model based on data such as lipid molecule chemical structure information, a transfection efficiency, and/or a cell survival rate can be generated, and lipid molecule chemical structure information, a transfection efficiency, and/or a cell survival rate can be inferred by using the learning model.

That is, the present disclosure provides the following.
[1] An inference device including:
   an acquiring unit configured to acquire input data including at least chemical structure information on a lipid molecule; and
   a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
   wherein the learned model infers a transfection efficiency and/or a cell survival rate associated with the input data newly acquired by the acquiring unit.
[2] The inference device as described in [1], wherein the transfection efficiency and/or the cell survival rate used when the learning process is performed are calculated based on a measurement result measured by introducing, into the cell, the active ingredient encapsulated in the particle containing the lipid molecule having the chemical structure information that is used when the learning process is performed.
[3] The inference device as described in [2], wherein the learned model is generated by updating model parameters of the learning model so that an output, obtained when the input data including at least the chemical structure information on the lipid molecule is input into the learning model, approaches the transfection efficiency and/or the cell survival rate calculated based on the measurement result.
[4] The inference device as described in [1],
   wherein the acquiring unit performs predetermined preprocessing on the newly acquired input data, and
   wherein the learned model infers the transfection efficiency and/or the cell survival rate associated with the preprocessed input data.
[5] An inference method including:
   an acquisition step of acquiring input data including at least chemical structure information on a lipid molecule; and
   an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
   wherein the execution step infers, by executing the learned model, a transfection efficiency and/or a cell survival rate associated with the input data newly acquired in the acquisition step.
[6] An inference program for causing a computer to perform:
   an acquisition step of acquiring input data including at least chemical structure information on a lipid molecule; and
   an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
   wherein the execution step infers, by executing the learned model, a transfection efficiency and/or a cell survival rate associated with the input data newly acquired in the acquisition step.
[7] A model generation method of generating a learned model by performing a learning process on a learning model that associates input data including at least chemical structure information on a lipid molecule with a transfection efficiency of an active ingredient encapsulated in a particle containing the lipid molecule into a cell and/or a cell survival rate.
[8] An inference device including:
   an acquiring unit configured to acquire input data including a precondition for designing or selecting a lipid molecule forming a particle encapsulating an active ingredient; and
   a learned model generated by performing a learning process on a learning model that associates input data including a precondition for designing or selecting a lipid molecule forming a particle encapsulating an active ingredient with chemical structure information on the lipid molecule,
   wherein the learned model infers chemical structure information on a lipid molecule associated with the input data newly acquired by the acquiring unit.
[9] The inference device as described in [8], wherein the input data used when the learning process is performed includes a transfection efficiency of the active ingredient encapsulated in the particle containing the lipid molecule into a cell and/or a cell survival rate, calculated based on a measurement result measured by introducing, into the cell, the active ingredient encapsulated in the particle containing the lipid molecule that is designed or selected.
[10] The inference device as described in [8], wherein the learned model is generated by updating model parameters of the learning model so that an output, obtained when the input data including the precondition is input into the learning model, approaches the chemical structure information on the lipid molecule used when the learning process is performed.
[11] The inference device as described in [8],
   wherein the acquiring unit performs predetermined preprocessing on the newly acquired input data, and
   wherein the learned model infers the chemical structure information on the lipid molecule associated with the preprocessed input data.
[12] An inference method including:
   an acquisition step of acquiring input data including a precondition for designing or selecting a lipid molecule forming a particle encapsulating an active ingredient; and
   an execution step of executing a learned model generated by performing a learning process on a learning model that associates input data including a precondition for designing or selecting a lipid molecule forming a particle encapsulating an active ingredient with chemical structure information on the lipid molecule,
   wherein the execution step infers, by executing the learned model, chemical structure information on a lipid molecule associated with the input data newly acquired in the acquisition step.
[13] An inference program for causing a computer to perform:
   an acquisition step of acquiring input data including a precondition for designing or selecting a lipid molecule forming a particle encapsulating an active ingredient; and
   an execution step of executing a learned model generated by performing a learning process on a learning model that associates input data including a precondition for designing or selecting a lipid molecule forming a particle encapsulating an active ingredient with chemical structure information on the lipid molecule,
   wherein the execution step infers, by executing the learned model, chemical structure information on a lipid molecule associated with the input data newly acquired in the acquisition step.
[14] A model generation method of generating a learned model by performing a learning process on a learning model that associates input data including a precondition for designing or selecting a lipid molecule forming a particle encapsulating an active ingredient with chemical structure information on the lipid molecule.
[15] An inference service providing system including:
   an acquiring unit configured to acquire, from a user, a precondition for designing or selecting a lipid molecule forming a particle encapsulating an active ingredient;
   a learned model generated by performing a learning process on a learning model that associates input data including a precondition for designing or selecting a lipid molecule forming a particle encapsulating an active ingredient with chemical structure information on the lipid molecule; and
   a providing unit configured to provide, to the user, chemical structure information on a lipid molecule inferred by the learned model by input data, including the precondition newly acquired by the acquiring unit from the user, being input.
[16] The inference service providing system as described in [15], further including a charging unit configured to charge the user when the learned model infers the chemical structure information on the lipid molecule by the input data, including the precondition newly acquired by the acquiring unit from the user, being input.
[17] The inference service providing system as described in [16], wherein the charging unit changes details of the charge applied to the user, when a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the learned model, are acquired by the user.
[18] An inference service providing method including:
   an acquisition step of acquiring, from a user, a precondition for designing or selecting a lipid molecule forming a particle encapsulating an active ingredient;
   an execution step of executing a learned model generated by performing a learning process on a learning model that associates input data including a precondition for designing or selecting a lipid molecule forming a particle encapsulating an active ingredient with chemical structure information on the lipid molecule; and
   a provision step of providing, to the user, chemical structure information on a lipid molecule inferred by the learned model by input data, including the precondition newly acquired in the acquisition step from the user, being input.
[19] An inference program for causing a computer to perform:
   an acquisition step of acquiring, from a user, a precondition for designing or selecting a lipid molecule forming a particle encapsulating an active ingredient;
   an execution step of executing a learned model generated by performing a learning process on a learning model that associates input data including a precondition for designing or selecting a lipid molecule forming a particle encapsulating an active ingredient with chemical structure information on the lipid molecule; and
   a provision step of providing, to the user, chemical structure information on a lipid molecule inferred by the learned model by input data including the precondition newly acquired in the acquisition step from the user being input.
[20] An inference device including:
   an acquiring unit configured to acquire input data including a precondition for designing or selecting a lipid molecule forming a particle encapsulating an active ingredient;
   a reinforcement learning model configured to infer, by the input data including the precondition acquired by the acquiring unit being input, chemical structure information on the lipid molecule; and
   a calculating unit configured to calculate a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated by the calculating unit.
[21] The inference device as described in [20], wherein the calculating unit calculates the reward such that the reward is maximized by the transfection efficiency and/or the cell survival rate being increased.
[22] The inference device as described in [20],
   wherein the acquiring unit performs predetermined preprocessing on the input data, and
   wherein the reinforcement learning model infers the chemical structure information on the lipid molecule by the preprocessed input data being input.
[23] An inference method including:
   an acquisition step of acquiring input data including a precondition for designing or selecting a lipid molecule forming a particle encapsulating an active ingredient;
   an execution step of executing a reinforcement learning model configured to infer, by the input data including the precondition acquired in the acquisition step being input, chemical structure information on the lipid molecule; and
   a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated in the calculation step.
[24] An inference program for causing a computer to perform:
   an acquisition step of acquiring input data including a precondition for designing or selecting a lipid molecule forming a particle encapsulating an active ingredient;
   an execution step of executing a reinforcement learning model configured to infer, by the input data including the precondition acquired in the acquisition step being input, chemical structure information on the lipid molecule; and
   a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated in the calculation step.
[25] An inference service providing system including:
   an acquiring unit configured to acquire, from a user, a precondition for designing or selecting a lipid molecule forming a particle encapsulating an active ingredient;
   a reinforcement learning model configured to infer, by input data including the precondition acquired by the acquiring unit from the user being input, chemical structure information on the lipid molecule;
   a providing unit configured to provide, to the user, the chemical structure information on the lipid molecule inferred by the reinforcement learning model; and
   a calculating unit configured to calculate a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated by the calculating unit.
[26] The inference service providing system as described in [25], further including a charging unit configured to charge the user when the providing unit provides, to the user, the chemical structure information on the lipid molecule that the reinforcement learning model infers.
[27] The inference service providing system as described in [26], wherein the charging unit changes details of the charge applied to the user when the transfection efficiency of the active ingredient encapsulated in the particle containing the lipid molecule into the cell and/or the cell survival rate, calculated based on the measurement result measured by introducing, into the cell, the active ingredient encapsulated in the particle containing the lipid molecule having the chemical structure information inferred by the reinforcement learned model, are acquired by the user.
[28] An inference service providing method including:
   an acquisition step of acquiring, from a user, a precondition for designing or selecting a lipid molecule forming a particle encapsulating an active ingredient;
   an execution step of executing a reinforcement learning model configured to infer, by input data including the precondition acquired in the acquisition step from the user being input, chemical structure information on the lipid molecule;
   a provision step of providing, to the user, the chemical structure information on the lipid molecule inferred by the reinforcement learning model; and
   a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated by the calculation step.
[29] An inference service providing program for causing a computer to perform:
   an acquisition step of acquiring, from a user, a precondition for designing or selecting a lipid molecule forming a particle encapsulating an active ingredient;
   an execution step of executing a reinforcement learning model configured to infer, by input data including the precondition acquired in the acquisition step from the user being input, chemical structure information on the lipid molecule;
   a provision step of providing, to the user, the chemical structure information on the lipid molecule inferred by the reinforcement learning model; and
   a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
   wherein the reinforcement learning model performs a learning process based on the reward calculated by the calculation step.
[30] An inference device including:
   a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
   a generating unit configured to repeat, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.
[31] The inference device as described in [30], wherein the generating unit generates the next new chemical structure information on the lipid molecule by selecting a search space from among a plurality of search spaces corresponding to combinations of a molecular fragment of formable hydrocarbons and a chemical structure of a lipid molecule, based on the inference result, and using a characteristic of the selected search space.
[32] The inference device as described in [31], wherein the plurality of search spaces are different from each other in terms of a combination of a length of the molecular fragment, a degree of saturation, a number of branches, and a type of the chemical skeleton of the lipid molecule.
[33] The inference device as described in [31], wherein the generating unit generates the next new chemical structure information on the lipid molecule under a predetermined constraint condition.
[34] The inference device as described in [30], further including an acquiring unit configured to acquire a precondition for designing or selecting a lipid molecule forming a particle encapsulating an active ingredient,
   wherein the generating unit generates the next new chemical structure information on the lipid molecule by using the acquired precondition as a constraint condition.
[35] An inference method including:
   an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
   a generation step of repeating, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.
[36] An inference program for causing a computer to perform:
   an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
   a generation step of repeating, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.

### Effects of the Invention

According to the present disclosure, an operation of designing or selecting a chemical structure of a lipid molecule forming a particle encapsulating an active ingredient can be supported.

### Brief Description of the Drawings

[FIG. 1] FIG. 1 is a diagram illustrating an accumulation example of various data in a drug delivery system examination process.
[FIG. 2] FIG. 2 is a diagram illustrating an application example of an inference device according to a first embodiment.
[FIG. 3] FIG. 3 is a diagram illustrating an example of a hardware configuration of the inference device.
[FIG. 4] FIG. 4 is a diagram illustrating an example of a functional configuration of a learning device according to the first embodiment.
[FIG. 5] FIG. 5 is a diagram illustrating an example of a functional configuration of the inference device according to the first embodiment.
[FIG. 6] FIG. 6 is an example of a flowchart illustrating a flow of a process of inferring a transfection efficiency and/or a cell survival rate.
[FIG. 7A] FIG. 7A is a diagram illustrating an example of the learning device.
[FIG. 7B] FIG. 7B is a diagram illustrating an example of the inference device.
[FIG. 8] FIG. 8 is a diagram illustrating an application example of an inference device according to a second embodiment.
[FIG. 9] FIG. 9 is a diagram illustrating an example of a functional configuration of a learning device according to the second embodiment.
[FIG. 10] FIG. 10 is a diagram illustrating an example of a functional configuration of the inference device according to the second embodiment.
[FIG. 11] FIG. 11 is an example of a flowchart illustrating a flow of a process of inferring lipid molecule chemical structure information.
[FIG. 12] FIG. 12 is a diagram illustrating an application example of an inference service providing system according to a third embodiment.
[FIG. 13] FIG. 13 is an example of a flowchart illustrating a flow of an inference service providing process.
[FIG. 14] FIG. 14 is a diagram illustrating an application example of an inference service providing system according to a fourth embodiment.
[FIG. 15] FIG. 15 is a diagram illustrating an example of a functional configuration of an inference device according to the fourth embodiment.
[FIG. 16] FIG. 16 is another example of the flowchart illustrating the flow of the inference service providing process.
[FIG. 17] FIG. 17 is a diagram illustrating an example of a functional configuration of an inference device according to a fifth embodiment.
[FIG. 18] FIG. 18 is an example of a flowchart illustrating a flow of a generation process.
[FIG. 19] FIG. 19 is a diagram illustrating an example of a functional configuration of an inference device according to a sixth embodiment.

### Embodiment for Carrying Out the Invention

In the following, embodiments will be described with reference to the accompanying drawings. Here, in the present specification and the drawings, components having substantially the same functional configuration are referenced by the same reference numerals, and duplicated description will be omitted.

### [First Embodiment]

### <Accumulation example of various data in a drug delivery system examination process>

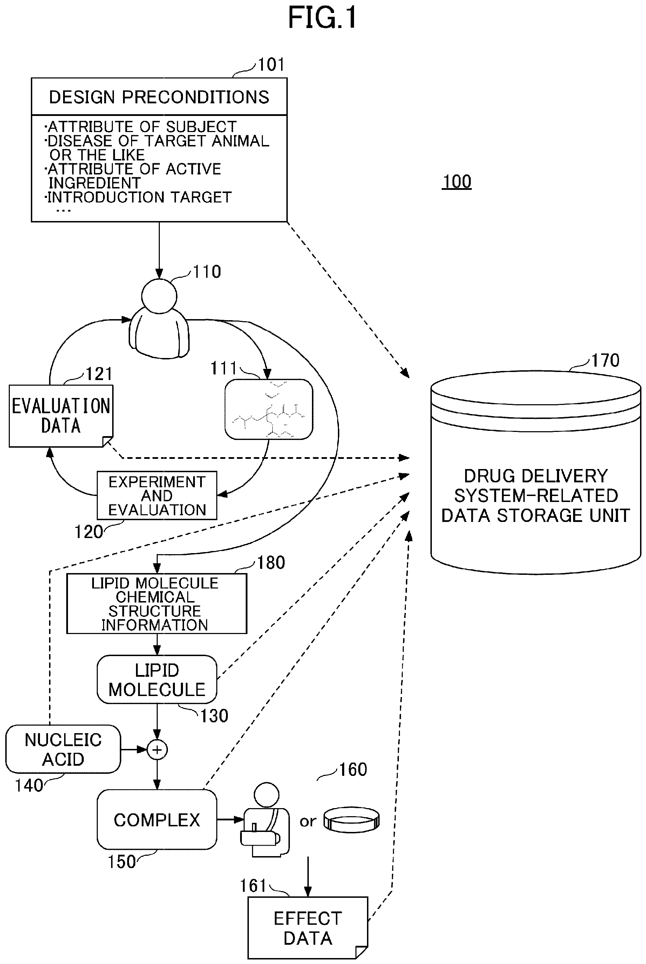

First, an accumulation example of various data in a general drug delivery system examination process will be described. FIG. 1 is a diagram illustrating the accumulation example of various data in the drug delivery system examination process.

As illustrated in FIG. 1, in a drug delivery system examination process 100, in designing (or selecting) a chemical structure of a lipid molecule forming a particle encapsulating an active ingredient, first, as preconditions for design or selection (hereinafter, simply referred to as "the design preconditions"), the following items (see the design preconditions 101) are input to a designer 110.
- Attribute of a subject (a human or non-human animal as a target (hereinafter, referred to as "a target animal or the like" in the present specification)) 160 or a cell and/or tissue 160 outside a living organism (in vitro, in situ, or ex vivo)
- Type of a disease of the target animal or the like 160
- Attribute(e.g., a type, chemical structure information, and the like on the active ingredient (e.g., the nucleic acid 140)) of an encapsulated active ingredient (e.g., a nucleic acid 140)
- Target into which a particle of a complex 150 is introduced (a specific cell in a living organism (in vivo) of the target animal or the like 160 or a specific cell 160 outside a living organism (in vitro, in situ, or ex vivo)) Here, the particle encapsulating the active ingredient at least indicates a concept including:
- a case in which lipid molecules are mixed together with an active ingredient (e.g., the nucleic acid 140) to form a complex particle; or
- a case in which lipid molecules form a shell and an active ingredient (e.g., the nucleic acid 140) is included in the shell to form a complex particle.

Examples of lipid molecules handled in one aspect of the present disclosure include cationic lipid molecules. A cationic lipid indicates a lipid that has a net positive charge at a selected pH, such as physiological pH. Examples of a method for producing lipid molecules and a particle encapsulating an active ingredient include methods described in WO 2016/021683, WO 2019/131839, WO 2020/032184, and the like. Examples of lipid molecules handled in another aspect of the present disclosure include anionic lipid molecules, cholesterol derivative molecules, and amphiphilic lipid molecules.

The nucleic acid handled in one aspect of the present disclosure may be any molecule in which a nucleotide and a molecule having a function equivalent to the function of the nucleotide are polymerized, and examples of the nucleic acid include RNA, which is a polymer of ribonucleotides, DNA, which is a polymer of deoxyribonucleotides, a polymer in which ribonucleotides and deoxyribonucleotides are mixed, and a nucleotide polymer containing a nucleotide analog, and may further be a nucleotide polymer containing a nucleic acid derivative. Additionally, the nucleic acid may be a single-stranded nucleic acid or a double-stranded nucleic acid. Additionally, the double-stranded nucleic acid includes a double-stranded nucleic acid in which one strand is hybridized with the other strand under a stringent condition. Additionally, the nucleic acid handled in the present embodiment is not particularly limited, and may be, for example, a nucleic acid for the purpose of improving a disease, a symptom, a disorder, or a pathological state, alleviating a disease, a symptom, a disorder, or a pathological state, preventing the onset thereof, or the like (in the present specification, may be referred to as "treatment of a disease or the like"), and may be a nucleic acid for regulating expression of a desired protein useful for research but not contributing to the treatment of a disease or the like. Specific examples of the nucleic acids handled in the present embodiment include siRNA, miRNA, miRNAmimic, antisense nucleic acids, ribozymes, mRNA, decoy nucleic acids, aptamers, DNA, and artificially modified analogues or derivatives thereof.

The designer 110 designs or selects a chemical structure of a lipid molecule from the design preconditions 101 based on experience and know-how obtained thus far. Experiment processing and evaluation processing are performed by an experimenter and evaluator 120 on a lipid molecule 111 having the chemical structure designed or selected by the designer 110, and evaluation data 121 is notified to the designer 110.

The designer 110 designs or selects again the chemical structure of the lipid molecule based on the notified evaluation data 121. Experiment processing and evaluation processing are performed again by the experimenter and evaluator 120 on a lipid molecule 111' (not illustrated) having the chemical structure designed or selected again by the designer 110, and evaluation data 121' (not illustrated) is notified to the designer 110.

The design or selection performed by the designer 110 and the experiment processing and evaluation processing performed by the experimenter and evaluator 120 are repeated multiple times. This enables the designer 110 to search for a chemical structure of a more appropriate lipid molecule forming the particle encapsulating the nucleic acid 140.

Subsequently, when a lipid molecule 130 is generated based on lipid molecule chemical structure information 180 on the lipid molecule searched by the designer 110, the nucleic acid 140 is encapsulated in the particle containing the generated lipid molecules 130, and the particle of the complex 150 is formed. Here, in addition to the nucleic acid 140, a component other than the lipid molecule and the nucleic acid may be contained in the particle of the complex 150 as necessary. Such a component includes, for example, stabilizers, antioxidants, and the like in appropriate amounts. These components may be pharmaceutically acceptable components.

The formed particle of the complex 150 is applied to the target animal or the like 160, or the cell and/or tissue 160 outside the living organism (in vitro, in situ, or ex vivo). A change caused by introducing the particle of the complex 150 into a specific cell in the living organism (in vivo) of the target animal or the like 160 (or a change caused by introducing the particle into a specific cell 160 outside the living organism (in vitro, in situ, or ex vivo)) is measured by various measuring methods and measuring devices, and is output as effect data 161.

Here, a method known to those skilled in the art may be used as a method of applying the particle of the complex 150 to the target animal or the like 160. Specific examples of the method of the application to the target animal or the like 160 include administration as a bolus or continuous injection over a certain period of time by intravenous, intramuscular, intraperitoneal, intracerobrospinal, subcutaneous, intra-articular, intrasynovial, intrathecal, oral, topical, or inhalation routes. Additionally, those skilled in the art can appropriately determine the number of administrations, the dose, and the administration interval. Specific examples of the application method to a cell and/or tissue outside the living organism (in vitro, in situ, or ex vivo) include adding a particle of the complex 150 to a container in which target cells are cultured and performing culture for a certain period of time. Those skilled in the art can appropriately determine the number of additions, the amount of an addition, the interval between additions, the culture condition, the culture period, and the like.

The effect data 161 includes the transfection efficiency of the nucleic acid 140 encapsulated in the particle containing the lipid molecule 130 into the cell and/or the cell survival rate, calculated from the measurement result.

The transfection efficiency can be appropriately evaluated using a publicly-known method, based on attributes of the nucleic acid, which is the active ingredient, and the like. For example, when the siRNA is used as the nucleic acid, the evaluation can be performed based on a knockdown rate of the expression of a gene targeted by the siRNA. More specifically, the evaluation can be performed by comparing an expression level of the gene (for example, the mRNA) in a group to which the particle containing the siRNA is administered (an administration group) with an expression level of the gene in a comparison group (for example, a group to which nothing is administered, a group to which the particle containing no siRNA is administered, or a group to which only a substance containing neither the siRNA nor the lipid molecule (for example, a group to which only physiological saline is administered or the like)), and by determining the ratio of the gene expression level in the administration group to the gene expression level in the comparison group. When the siRNA is used, it can be determined that as the ratio decreases, the transfection efficiency increases. Additionally, when the mRNA is used as the nucleic acid, the evaluation can be performed based on an expression level of a protein encoded by the mRNA. More specifically, the evaluation can be performed by comparing expression levels of proteins in substantially the same manner as in the case of using siRNA. When the mRNA is used, it can be determined that as the ratio increases, the transfection efficiency increases. Methods and devices for measuring gene expression levels, protein expression levels, and the like can be appropriately selected by those skilled in the art.

The cell survival rate when the transfection is performed can also be appropriately evaluated using a publicly-known method. For example, the evaluation can be performed by comparing the number of cells before the application with the number of cells after the application, and measuring the ratio of the number of cells after the application to the number of cells before the application. Methods and devices for measuring the number of cells can be appropriately selected by those skilled in the art.

As the evaluation data and effect data, the transfection efficiency into a cell and the cell survival rate are described as examples. Further, data related to the disposition within a living organism (absorption, distribution, and metabolism) and toxicity of the nucleic acid 140 in a living organism when the particle of the complex 150 is applied to the target animal or the like 160 can be included, and the evaluation data and effect data are not limited thereto.

Various data acquired in a sequence flow of the drug delivery system examination process 100 are accumulated in a drug delivery system-related data storage unit 170. As illustrated in FIG. 1, various data accumulated in the drug delivery system-related data storage unit 170 include, for example:
- design precondition 101;
- evaluation data 121;
- chemical structure information on the lipid molecule 130;
- type and chemical structure information about the nucleic acid 140
- chemical structure information on the complex 150;
- effect data 161 (including the transfection efficiency and/or the cell survival rate).

The chemical structure information on the lipid molecule is not particularly limited, and examples thereof include a chemical formula, a three dimensional structure, a molecular weight, the number of carbon atoms, the number of nitrogen atoms, the number of oxygen atoms, an electric charge, and the like.

The chemical structure information on the nucleic acid is not particularly limited, and examples thereof include the number of respective bases constituting the nucleic acid, a chemical formula, a three dimensional structure, a molecular weight, an electric charge, and the like.

Examples of the chemical structure information on the complex include a particle diameter, a membrane potential, and the like of the complex.

The chemical structure information can be appropriately measured using a publicly-known method.

Here, various public information (for example, patent publications and papers) and data available from databases may be further included in various data accumulated in the drug delivery system-related data storage unit 170.

### <Application example of the inference device>

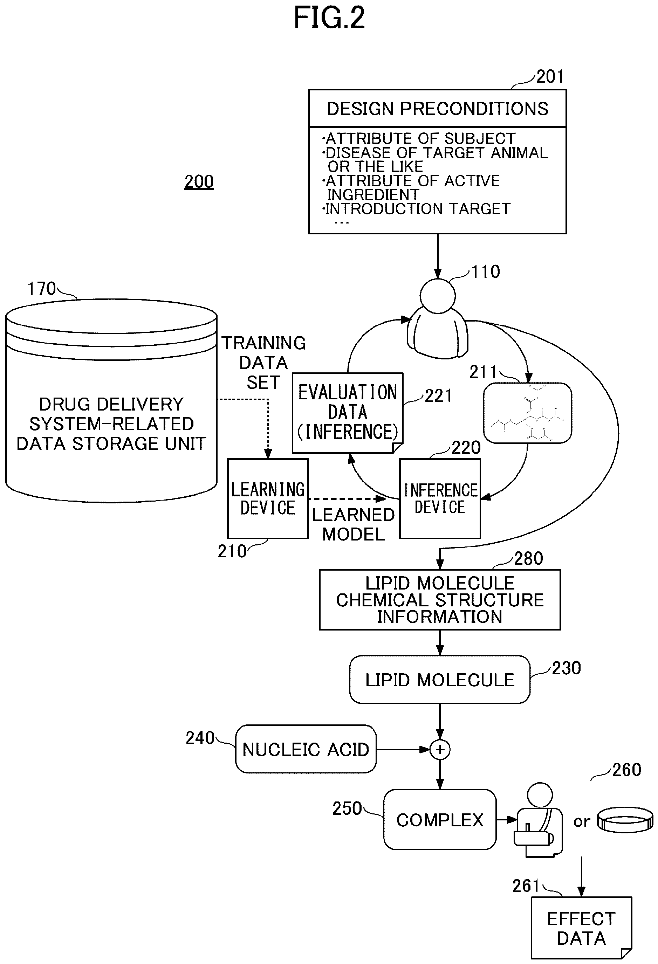

Next, an application example of an inference device to the drug delivery system examination process when the inference device is generated using various data stored in the drug delivery system-related data storage unit 170 illustrated in FIG. 1 will be described. FIG. 2 is a diagram illustrating an application example of an inference device according to a first embodiment.

As in FIG. 1, in a drug delivery system examination process 200 to which an inference device 220 according to the first embodiment is applied, when designing (or selecting) the chemical structure of the lipid molecule forming the particle encapsulating the active ingredient, the following and the like are input to the designer 110, as design preconditions 201.
- Attribute of the subject (a target animal or the like 260 or a cell and/or tissue 260 outside the living organism (in vitro, in situ, or ex vivo)
- Type of a disease of the target animal or the like 260
- Attribute of the encapsulated active ingredient (e.g., the nucleic acid 240) (e.g., a type, the chemical structure information, and the like about the active ingredient (e.g., the nucleic acid 240))
- Target into which a particle of a complex 250 is introduced (a specific cell in the living organism (in vivo) of the target animal or the like 260 or a specific cell 260 outside the living organism (in vitro, in situ, or ex vivo)

The designer 110 designs or selects the chemical structure of the lipid molecule from the design preconditions 201 based on experience and know-how obtained thus far. The chemical structure information on the lipid molecule 211 having the chemical structure designed or selected by the designer 110 is input to the inference device 220.

Here, the inference device 220 includes a learned model generated by a learning device 210. The learning device 210 generates the learned model by performing a learning process on a learning model by using a training data set generated based on various data accumulated in the drug delivery system-related data storage unit 170.

The inference device 220 uses the learned model generated by the learning device 210 to generate evaluation data 221 related to the chemical structure information on the lipid molecule 211. The evaluation data 221 generated by the inference device 220 is notified to the designer 110.

The design or selection performed by the designer 110 and the generation of evaluation data performed by the inference device 220 are repeated multiple times. This enables the designer 110 to search for a chemical structure of a more appropriate lipid molecule forming the particle encapsulating the nucleic acid 240.

Subsequently, when a lipid molecule 230 is generated based on lipid molecule chemical structure information 280 searched by the designer 110, the nucleic acid 240 is encapsulated in the particle containing the generated lipid molecule 230 to form the particle of the complex 250. Here, in addition to the nucleic acid 240, a component other than the lipid molecule and the nucleic acid may be included in the particle of the complex 250 as necessary. Such a component includes, for example, stabilizers, antioxidants, and the like in appropriate amounts. These components may be pharmaceutically acceptable components.

The formed particle of the complex 250 is applied to the target animal or the like 260, the cell and/or tissue 260 outside the living organism (in vitro, in situ, or ex vivo), or the like. A change caused by introducing the particle of the complex 250 into a specific cell in the living organism (in vivo) of the target animal or the like 260 (or a change caused by introducing the particle into the specific cell 260 outside the living organism (in vitro, in situ, or ex vivo) is measured by various measuring methods and measuring devices, and is output as effect data 261.

Here, a method publicly known to those skilled in the art may be used as a method of applying the particle of the complex 250 to the target animal or the like 260. Specific examples of the method of the application to the target animal or the like 260 include administration as a bolus or continuous injection over a certain period of time by intravenous, intramuscular, intraperitoneal, intracerobrospinal, subcutaneous, intra-articular, intrasynovial, intrathecal, oral, topical, or inhalation routes. Additionally, those skilled in the art can appropriately determine and design the number of administrations, the dose, and the administration interval. Specific examples of the application method to a cell and/or tissue outside the living organism (in vitro, in situ, or ex vivo) include adding a particle of the complex 250 to a container in which target cells are cultured and performing culture for a certain period of time. Those skilled in the art can appropriately determine the number of additions, the amount of an addition, the interval between additions, the culture condition, the culture period, and the like.

Additionally, a change caused by introducing the particle of the complex 250 into a specific cell or the like in the living organism (in vivo) of the target animal or the like 260 can be captured by measuring the target animal or the like 260 into which the particle of the complex 250 have been introduced or by measuring a sample containing the specific cell obtained from the target animal or the like 260. The sample is not particularly limited as long as the sample contains the specific cell, and examples thereof include whole blood, blood plasma, urine, serum, lymph, saliva, anal and vaginal secretions, sweat, semen, body fluids that are not limited by these, and a tissue sample or a cell obtained from biopsy of an organ or a tissue. Additionally, in order to provide the obtained sample for measurement, the sample may be labeled by any publicly-known method.

Additionally, examples of the "target animal and the like" include, in addition to humans, animals such as mice, rats, guinea pigs, dogs, cats, rabbits, cows, horses, sheep, goats, and pigs, but is not limited thereto. The target animal or the like may be a healthy human or animal, or a human (patient) or animal suffering from any disease.

Furthermore, any in vitro or in vivo method known to those skilled in the art may be used as the measurement method. Specific examples thereof include flow cytometry, immunological assay, mRNA transcript analysis, a PCR method, a hybridization method, and the like. Alternatively, the examples thereof include a sequencing method, an RFLP method, Western blot, ELISA, radioimmunoassay, immunoprecipitation, FACS, HPLC, surface plasmon resonance, optical spectroscopy, mass spectrometry, and the like. In the following, the transfection efficiency into a cell and the cell survival rate are described as examples of the measurement results, but the measurement items are not limited thereto.

The effect data 261 includes the transfection efficiency of the nucleic acid 240 encapsulated in the particle containing the lipid molecule 230 into the cell and/or the cell survival rate, calculated from the measurement result. Further, as the effect data 261, data related to the disposition within a living organism (absorption, distribution, and metabolism) and toxicity of the nucleic acid 240 in a living organism when the particle of the complex 250 is applied to the target animal or the like 260 can be included, but the effect data 261 is not limited thereto.

As described above, conventionally, the evaluation data 221 is generated by the experimenter and the evaluator 120 performing the experiment processing and the evaluation processing. However, by applying the inference device 220, the evaluation data 121 can be generated without performing the experiment processing and the evaluation processing.

This can shorten the time required for searching for a chemical structure of a more appropriate lipid molecule forming the particle encapsulating the nucleic acid 240. That is, according to the inference device 220 in the first embodiment, the operation of designing or selecting the chemical structure of the lipid molecule forming the particle encapsulating the nucleic acid can be supported.

### <Hardware configuration of the learning device and the inference device>

Next, a hardware configuration of the learning device 210 and the inference device 220 will be described. Here, because the learning device 210 and the inference device 220 have substantially the same hardware configuration, the hardware configuration of the inference device 220 will be described here.

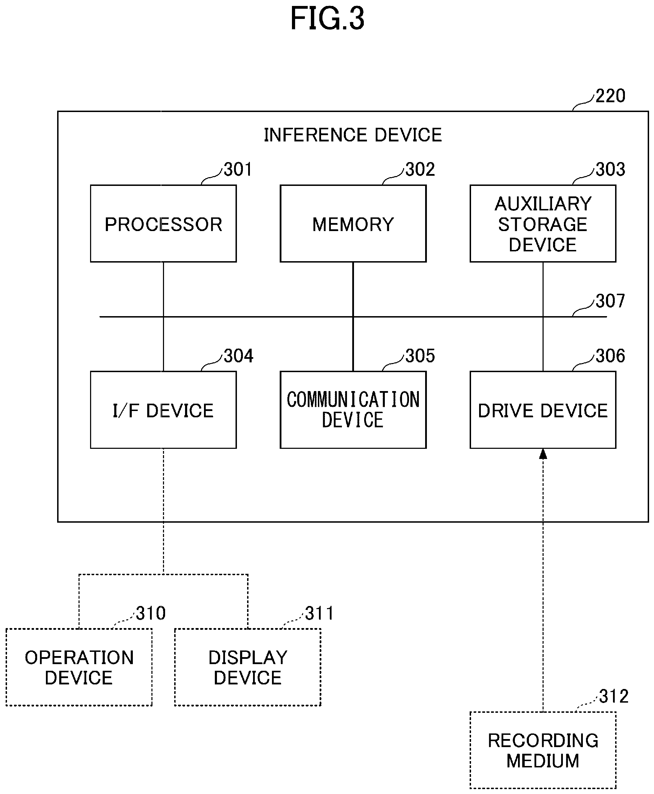

FIG. 3 is a diagram illustrating an example of the hardware configuration of the inference device. As illustrated in FIG. 3, the inference device 220 includes a processor 301, a memory 302, an auxiliary storage device 303, an interface (I/F) device 304, a communication device 305, and a drive device 306. Here, respective hardware components of the inference device 220 are connected to each other via a bus 307.

The processor 301 includes various arithmetic devices such as a central processing unit (CPU) and a graphics processing unit (GPU). The processor 301 reads out various programs installed in the auxiliary storage device 303 to the memory 302 and executes the programs.

The memory 302 includes a main storage device such as a read only memory (ROM) and a random access memory (RAM). The processor 301 and the memory 302 form what is called a computer, and the computer achieves various functions by the processor 301 executing various programs read out to the memory 302.

The auxiliary storage device 303 stores various programs and various data used when the various programs are executed by the processor 301.

The I/F device 304 is a connection device that connects the operation device 310 and the display device 311 to the inference device 220. The I/F device 304 receives various instructions to the inference device 220 via the operation device 310. Additionally, the I/F device 304 outputs a processing result of the inference device 220 via the display device 311.

The communication device 305 is a communication device for communicating with another device via a network.

The drive device 306 is a device for setting the recording medium 312. The recording medium 312 described here includes a medium for optically, electrically, or magnetically recording information, such as a CD-ROM, a flexible disk, or a magneto-optical disk. Additionally, the recording medium 312 may include a semiconductor memory or the like that electrically records information, such as a ROM or a flash memory.

Here, the various programs installed in the auxiliary storage device 303 are installed by, for example, the distributed recording medium 312 being set in the drive device 306 and reading out the various programs recorded in the recording medium 312 by the drive device 306. Alternatively, the various programs to be installed in the auxiliary storage device 303 may be installed by being downloaded from a network via the communication device 305.

### <Functional configuration of the learning device>

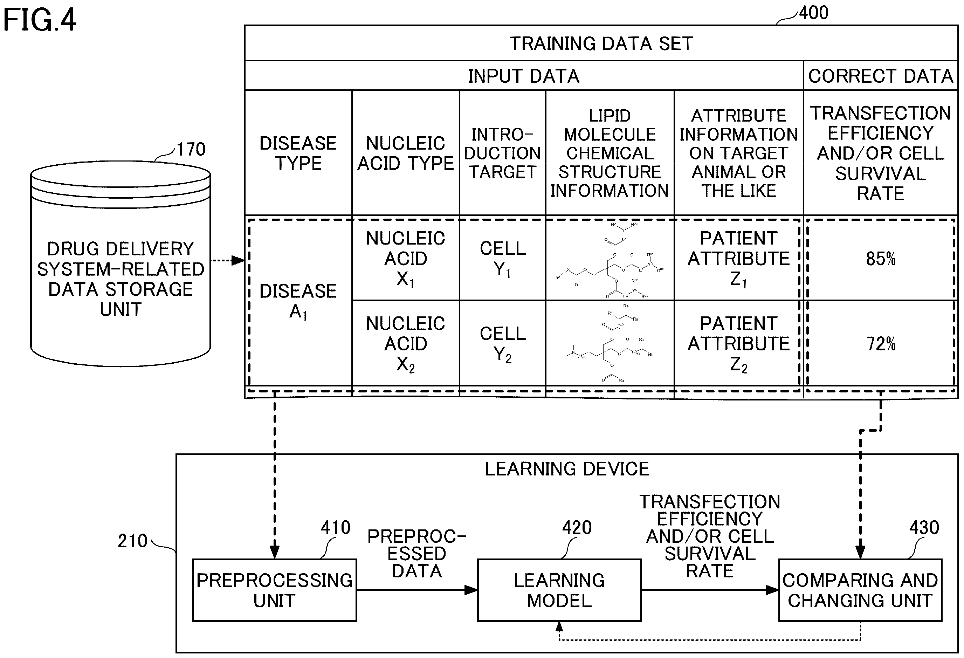

Next, a functional configuration of the learning device 210 will be described in detail. FIG. 4 is a diagram illustrating an example of the functional configuration of the learning device according to the first embodiment. In FIG. 4, a training data set 400 is an example of the training data set generated based on various data stored in the drug delivery system-related data storage unit 170. Here, although information items of the training data (input data and correct data) are exemplified below, the embodiment is not limited thereto, and a part or all of the items can be appropriately used as the training data.

As illustrated in FIG. 4, the training data set 400 includes the input data and the correct data, and the input data includes, for example, a "disease type", a "nucleic acid type", an "introduction target", "chemical structure information on the lipid molecule", and "attribute information on the target animal or the like" as information items.

In the "disease type", for example, when the target animal or the like 160 is a patient (hereinafter, may be referred to as a "target patient" in the present specification), a "disease A₁", which is a type of the disease thereof, is stored.

In the "nucleic acid type", for example, a "nucleic acid X₁", a "nucleic acid X₂", and the like are stored as types of the nucleic acids corresponding to the "disease A₁".

In the "introduction target", information indicating a target cell into which the particle of the complex is to be introduced is stored for each of the "disease type" and the "nucleic acid type". The example of FIG. 4 indicates that when the disease type = the "disease A₁" and the nucleic acid type = the "nucleic acid X₁", the particle of the complex is introduced into a "cell Y₁", and when the disease type = the "disease A₁" and the nucleic acid type = the "nucleic acid X₂", the particle of the complex is introduced into a "cell Y₂".

Examples of the information indicating the target cell here include an organ or tissue in which the origin or the cell is present in a living organism, the type of the cell (e.g., a nerve cell, a parenchymal cell, a stromal cell, or the like), and the like, but are not limited thereto.

In the "lipid molecule chemical structure information", information indicating a chemical structure of a more appropriate lipid molecule designed or selected in the past by the designer 110 based on the design preconditions 101, such as the "disease type", the "nucleic acid type", and the "introduction target", is stored.

In the "attribute information on the target animal or the like", the attribute information on the target animal or the like 160 (for example, the target patient) is stored. The example of FIG. 4 indicates that the particle of the complex, formed by the nucleic acid type = the "nucleic acid X₁" being encapsulated in the particle containing the lipid molecules, is introduced into the "cell Y₁" of the target patient having attribute information on the target animal or the like = a "patient attribute Z₁". Additionally, the example of FIG. 4 indicates that the particle of the complex, formed by the nucleic acid type = the "nucleic acid X₂" being encapsulated by the particle containing the lipid molecules, is introduced into the "cell Y₂" of the target patient having the attribute information on the target animal or the like = a "patient attribute Z₂".

With respect to the above, in the correct data, the "transfection efficiency and/or cell survival rate" are stored as the information item.

In the "transfection efficiency and/or cell survival rate", the transfection efficiency of the nucleic acid encapsulated in the particle containing the lipid molecule into a cell and/or the cell survival rate are stored. Specifically, for example, the transfection efficiency and/or the cell survival rate, calculated from a measurement result obtained by applying the particle of a corresponding complex to a corresponding target animal or the like 260 or the cell and/or tissue 260 outside the living organism (in vitro, in situ, or ex vivo) and measuring the applied target animal or the like, or the sample separated therefrom, are stored.

These training data sets may be data obtained from various public information (for example, patent publications and papers) or databases.

The example of FIG. 4 indicates that the transfection efficiency into the cell of the nucleic acid type = the "nucleic acid X₁" is "85%". Additionally, the example of FIG. 4 indicates that the transfection efficiency into the cell of the nucleic acid type = the "nucleic acid X₂" is "72%".

With respect to the above, a learning program is installed in the learning device 210, and by executing the learning program, the learning device 210 functions as a preprocessing unit 410, a learning model 420, and a comparing and changing unit 430.

The preprocessing unit 410 acquires "input data" of the training data set 400 and performs various preprocessing to generate preprocessed data suitable for being input into the learning model 420. The various preprocessing performed by the preprocessing unit 410 include processing for normalizing the input data, processing for vectorizing the input data, and the like.

The learning model 420 is a model that associates the input data with the correct data (the transfection efficiency and/or the cell survival rate). Specifically, the learning model 420 receives the preprocessed data notified by the preprocessing unit 410 as an input and outputs the transfection efficiency and/or the cell survival rate.

Here, a learning process of updating model parameters is performed on the learning model 420 by backpropagating the error calculated by the comparing and changing unit 430. This generates a learned model. That is, the learned model is generated by updating the model parameters of the learning model 420 so that the output of the learning model 420 approaches the correct data (the transfection efficiency and/or the cell survival rate).

The comparing and changing unit 430 calculates the error by comparing the transfection efficiency and/or the cell survival rate output from the learning model 420 with the correct data (the transfection efficiency and/or the cell survival rate) of the training data set 400. Additionally, the comparing and changing unit 430 updates the model parameters of the learning model 420 by backpropagating the calculated error.

### <Functional configuration of the inference device>

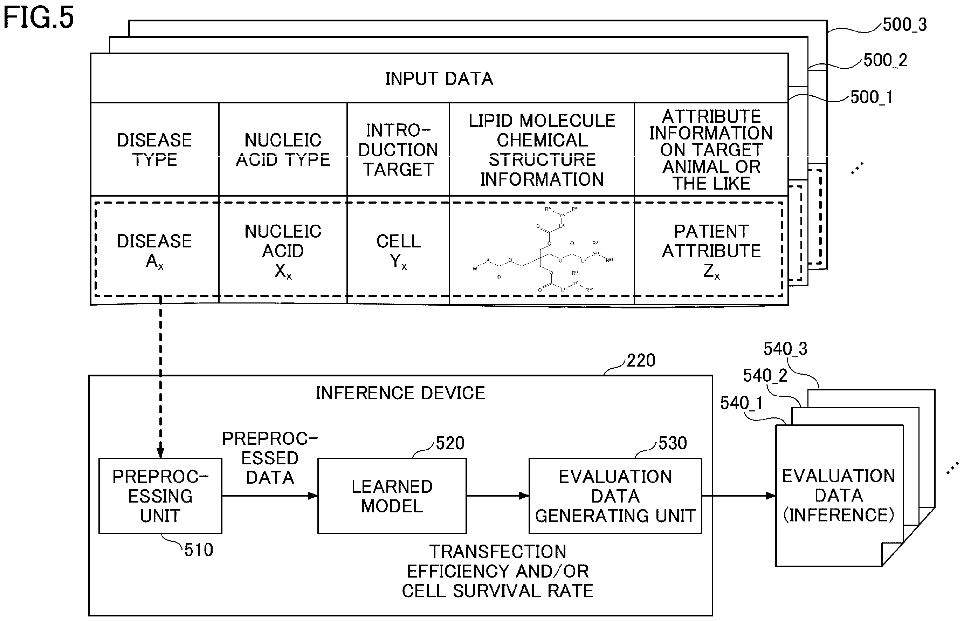

Next, a functional configuration of the inference device 220 will be described in detail. FIG. 5 is a diagram illustrating an example of the functional configuration of the inference device according to the first embodiment. As illustrated in FIG. 5, input data 500_1, 500_2, 500_3, ... include:
- information included in the design preconditions 201 (the disease type, the nucleic acid type, the introduction target, the attribute information on the target animal or the like, and the like); and
- chemical structure information on the lipid molecule designed or selected by the designer 110 based on the design preconditions 201.

### The input data may be data obtained from various public information (for example, patent publications and papers) or databases.

The input data 500_1, 500_2, 500_3, ... including the chemical structure information on the lipid molecules different from each other are input to the inference device 220. An inference program is installed in the inference device 220, and by executing the inference program, the inference device 220 functions as a preprocessing unit 510, a learned model 520, and an evaluation data generating unit 530.

The preprocessing unit 510 is an example of an acquiring unit and has the same function as that of the preprocessing unit 410 included in the learning device 210. Specifically, the preprocessing unit 510 acquires the input data 500_1, 500_2, 500_3, ... and performs various preprocessing on the acquired input data 500_1, 500_2, 500_3, ... to generate preprocessed data.

The learned model 520 is a learned model generated by the learning device 210 performing the learning process, receives the preprocessed data notified by the preprocessing unit 510 as an input, and infers the transfection efficiency and/or the cell survival rate.

The evaluation data generating unit 530 generates evaluation data 540_1, 540_2, 540_3, ... based on the transfection efficiency and/or the cell survival rate inferred by the learned model 520.

As described above, according to the inference device 220, the evaluation data can be generated by inferring the transfection efficiency and/or the cell survival rate without performing the experiment process and the evaluation process on the lipid molecule having the chemical structure designed or selected by the designer 110.

This can shorten the time required for searching for a chemical structure of a more appropriate lipid molecule forming the particle encapsulating the nucleic acid 240. That is, according to the inference device 220 of the first embodiment, the operation of designing or selecting the chemical structure of the lipid molecule forming the particle encapsulating the nucleic acid can be supported.

### <Flow of a process of inferring the transfection efficiency and/or the cell survival rate>

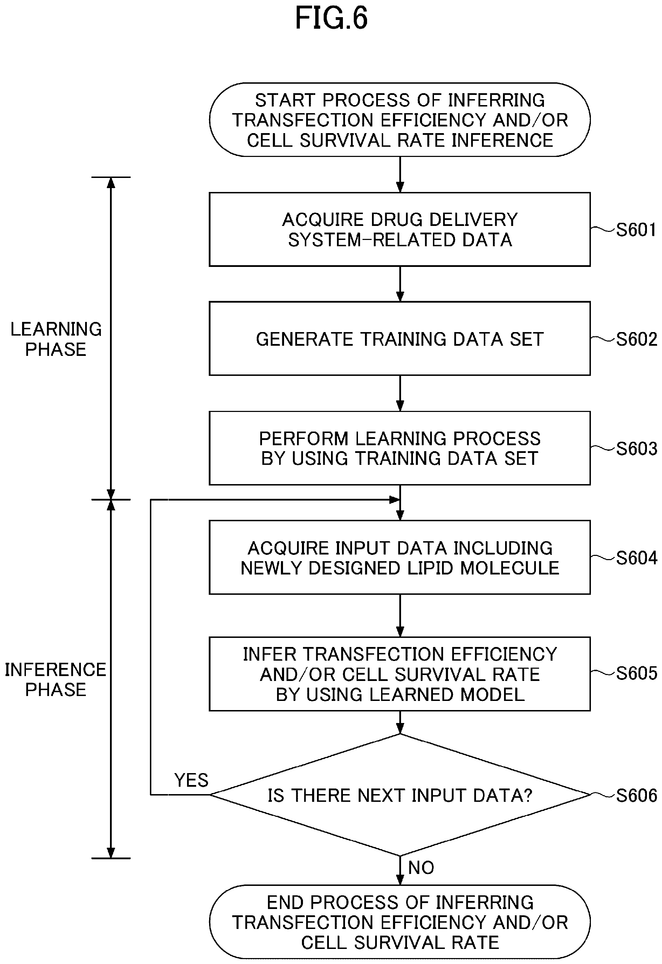

Next, a flow of a process of inferring the transfection efficiency and/or the cell survival rate in the drug delivery system examination process 200 will be described. FIG. 6 is an example of a flowchart illustrating the flow of the process of inferring the transfection efficiency and/or the cell survival rate.

In step S601, the learning device 210 acquires various data from the drug delivery system-related data storage unit 170.

In step S602, the learning device 210 generates the training data set 400 by using the acquired various data.

In step S603, the learning device 210 performs the learning process on the learning model 420 by using the training data set 400 to generate the learned model 520.

In step S604, the inference device 220 acquires the input data including the chemical structure information on the lipid molecule newly designed or selected by the designer 110 (for example, the input data 500_1).

In step S605, the inference device 220 executes the learned model 520 by inputting the acquired input data (for example, the input data 500_1) into the learned model 520, and infers the transfection efficiency and/or the cell survival rate. Additionally, the inference device 220 generates the evaluation data (for example, the evaluation data 540_1) based on the inferred transfection efficiency and/or cell survival rate.

In step S606, the inference device 220 determines whether there is next input data (for example, the input data 500_2, 500_3, ...).

If it is determined in step S606 that there is next input data (YES in step S606), the process returns to step S604.

Conversely, when it is determined in step S606 that there is no next input data (NO in step S606), the process of inferring the transfection efficiency and/or the cell survival rate is ended.

### <Summary>

As is apparent from the above description, the inference device 220 according to the first embodiment acquires the input data including at least the chemical structure information on the lipid molecule, and includes the learned model generated by performing the learning process on the learning model that associates the input data with the transfection efficiency into the cell of the nucleic acid encapsulated by the particle containing the lipid molecules and/or the cell survival rate. The learned model infers the transfection efficiency and/or the cell survival rate associated with newly acquired input data.

With this, according to the inference device 220 in the first embodiment, the time required to search for a chemical structure of a more appropriate lipid molecule forming the particle encapsulating the nucleic acid can be shortened. That is, according to the inference device 220 in the first embodiment, the operation of designing or selecting the chemical structure of the lipid molecule forming the particle encapsulating the nucleic acid can be supported.

### «Example 1»

In the following, specific examples of the learning device 210 and the inference device 220 according to the first embodiment will be described. Here, the following examples are merely examples, and the learning device 210 and the inference device 220 according to the first embodiment are not limited to the following examples.

### <Example of the learning device>

First, the example of the learning device 210 will be described. In the present example, the training data set is divided into a data set for learning and a data set for verification, and a learning process is performed on a learning model by using the data set for learning, to generate a learned model. Additionally, in the present example, the inference accuracy of the inference device 220 is evaluated by using the data set for verification.

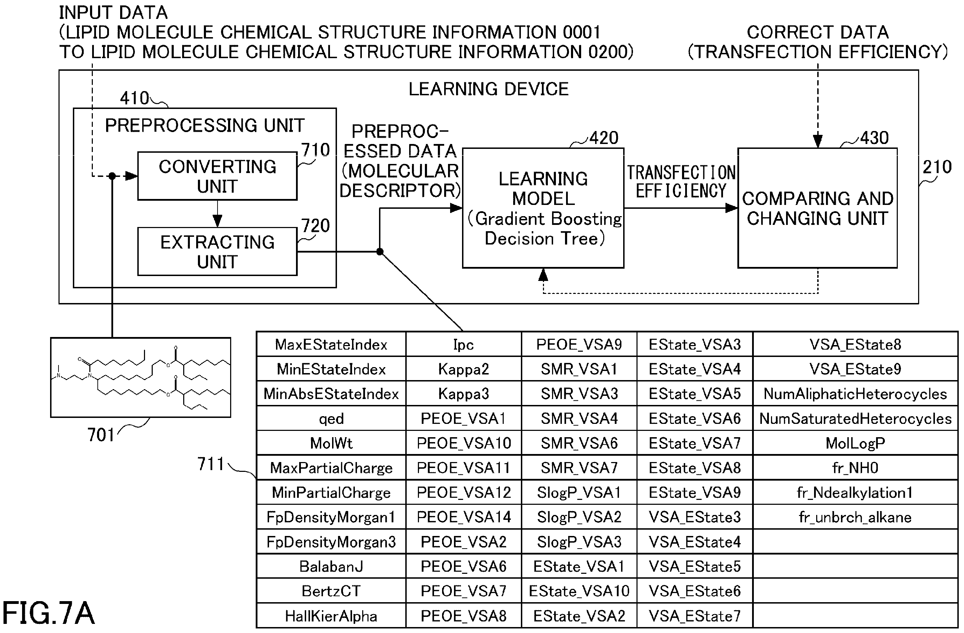

FIG. 7A is a diagram illustrating the example of the learning device. As illustrated in FIG. 7A, the preprocessing unit 410 includes a converting unit 710, and the converting unit 710 reads out input data from the data set for learning and converts the input data into a molecular descriptor. The example of FIG. 7A indicates a state in which 75 pieces of the lipid molecule chemical structure information are read out from among lipid molecule chemical structure information 0001 to lipid molecule chemical structure information 0200, and the readout chemical structure information is converted into 200 molecular descriptors. Here, in the example illustrated in FIG. 7A, the reference numeral 701 refers to one lipid molecule chemical structure information among 75 pieces of the lipid molecule chemical structure information read out by the converting unit 710.

Additionally, as illustrated in FIG. 7A, the preprocessing unit 410 includes an extracting unit 720 and deletes a molecular descriptor that is not appropriate for use in the learning process among the 200 molecular descriptors. Specifically, the extracting unit 720 deletes a molecular descriptor having a small variance among the 200 molecular descriptors, and further deletes a molecular descriptor that is found to be collinear. This enables the extracting unit 720 to extract a molecular descriptor suitable for use in the learning process. Here, in FIG. 7A, the reference numeral 711 is an example of the molecular descriptor extracted by the extracting unit 720, which is input to the learning model 420 as the preprocessed data.

Additionally, as illustrated in FIG. 7A, in the present example, it is assumed that the learning model 420 uses "Gradient Boosting Decision Tree" as a machine learning algorithm, and the hyperparameters are optimized by K-fold cross validation.

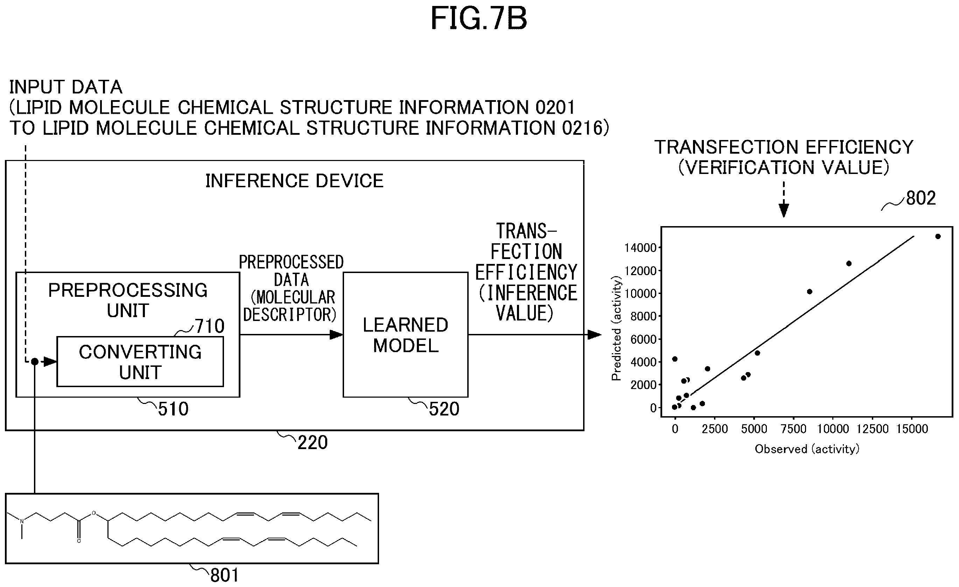

Additionally, as illustrated in FIG. 7A, in the present example, the learning model 420 performs the learning process by using the transfection efficiency as the correct data. As a result, the learning process is performed on the learning model 420 so as to associate the preprocessed data (the molecular descriptors) with the transfection efficiencies to generate the learned model 520 (see FIG. 7B).

### <Example of the inference device>

Next, the example of the inference device 220 will be described. FIG. 7B is a diagram illustrating the example of the inference device. As illustrated in FIG. 7B, the preprocessing unit 510 includes the converting unit 710, and the converting unit 710 reads out the input data from the data set for verification and converts the read input data into a molecular descriptor. The example in FIG. 7B indicates a state in which the converting unit 710 reads out 16 pieces of the lipid molecule chemical structure information from lipid molecule chemical structure information 0201 to lipid molecule chemical structure information 0216, and converts the read lipid molecule chemical structure information into 200 molecular descriptors. Here, in the example of FIG. 7B, the reference numeral 801 refers to one lipid molecule chemical structure information among 16 pieces of the lipid molecule chemical structure information read by the converting unit 710.

Additionally, as illustrated in FIG. 7B, the molecular descriptors converted by the converting unit 710 are input into the learned model 520 as the preprocessed data, and the learned model 520 infers transfection efficiencies.

In FIG. 7B, the reference numeral 802 is a result of evaluating the inference accuracy of the learned model 520 with respect to 16 pieces of the lipid molecule chemical structure information included in the data set for verification. Specifically, the reference numeral 802 indicates a state in which the inferred value and the measured value of the transfection efficiency are compared, and the inference accuracy is evaluated using the correlation coefficient and the mean absolute error as the indices. As indicated by the reference numeral 802, in the present example, it is found that the learned model 520 can infer the transfection efficiencies of 16 pieces of the lipid molecule chemical structure information with high inference accuracy.

### [Second embodiment]

Next, a second embodiment will be described. In the first embodiment described above, a case in which based on various data accumulated in the drug delivery system-related data storage unit 170, the learning device 210 generates the learned model that associates the following items has been described.
- Input data including the lipid molecule chemical structure information
- Transfection efficiency and/or the cell survival rate

With respect to the above, in the second embodiment, a case in which based on various data accumulated in the drug delivery system-related data storage unit 170, the learning device generates the learned model that associates the following items will be described.
- Information included in the design preconditions
- Lipid molecule chemical structure information In the following, the second embodiment will be described, focusing on differences from the first embodiment.

### <Application example of the inference device>

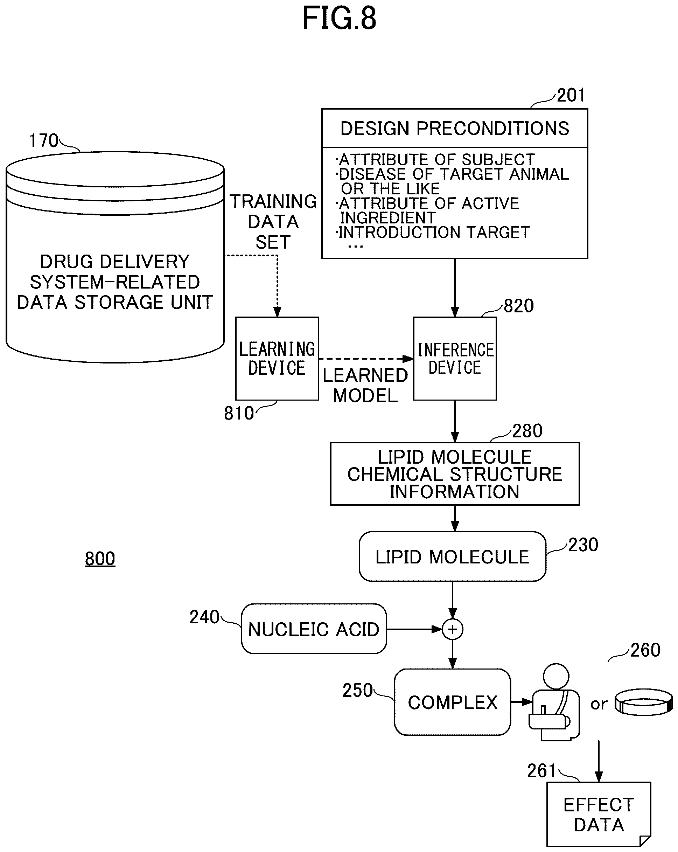

First, an application example of the inference device according to the second embodiment to the drug delivery system will be described. FIG. 8 is a diagram illustrating the application example of the inference device according to the second embodiment.

As illustrated in FIG. 8, in a drug delivery system examination process 800, when inferring chemical structure information on a more appropriate lipid molecule forming particle encapsulating the nucleic acid 240, the design preconditions 201 (specifically, input data including information included in the design preconditions 201) are input to an inference device 820.

Here, the inference device 820 includes a learned model generated by a learning device 810. The learning device 810 generates the learned model by performing a learning process on a learning model by using a training data set generated based on various data accumulated in the drug delivery system-related data storage unit 170.

The inference device 820 executes the learned model generated by the learning device 810 and infers the lipid molecule chemical structure information 280 from (the input data including) the information included in the design preconditions 201.

Subsequently, in the drug delivery system examination process 800, the lipid molecule 230 is generated based on the chemical structure information 280 inferred by the inference device 820, and the nucleic acid 240 is encapsulated in the particle containing the generated lipid molecule 230 to form the particle of the complex 250.

The formed particle of the complex 250 is applied to the target animal or the like 260, the cell and/or tissue 260 outside the living organism (in vitro, in situ, or ex vivo), or the like. A change caused by introducing the particle of the complex 250 into a specific cell in the living organism (in vivo) of the target animal or the like 260 (or a change caused by introducing the particle into a specific cell 260 outside the living organism (in vitro, in situ, or ex vivo)) is measured by various measuring methods and measuring devices, and is output as the effect data 261. As a method of applying the particle of the complex 250 to the target animal or the like 260 or to the cell and/or tissue outside the living organism (in vitro, in situ, or ex vivo), a method substantially the same as that in the [first embodiment] described above can be used.

In the effect data 261, the transfection efficiency of the nucleic acid 240 encapsulated in the particle containing the lipid molecule 230 into the cell and/or the cell survival rate calculated from the measurement result are included. The transfection efficiency and the cell survival rate can be evaluated in substantially the same manner as in the [First embodiment] described above.

As the effect data 261, the transfection efficiency into the cell and the cell survival rate are exemplified, but data related to the disposition within the living organism (absorption, distribution, and metabolism) and toxicity of the nucleic acid 240 in the living organism when the particle of the complex 250 is applied to the target animal or the like 260 may be further included. The effect data 261 are not limited thereto.

As described above, conventionally, the design or selection of the chemical structure of the lipid molecule is dependent on the experience or know-how of the designer 110, but according to the inference device 820, the lipid molecule chemical structure information 280 can be directly inferred from the information included in the design preconditions 201.

With this, a chemical structure of a more appropriate lipid molecule forming the particle encapsulating the nucleic acid 240 can be designed or selected without relying on the experience or know-how of the designer 110. That is, according to the inference device 820 in the second embodiment, the operation of designing or selecting the chemical structure of the lipid molecule forming the particle encapsulating the nucleic acid can be supported.

### <Functional configuration of the learning device>

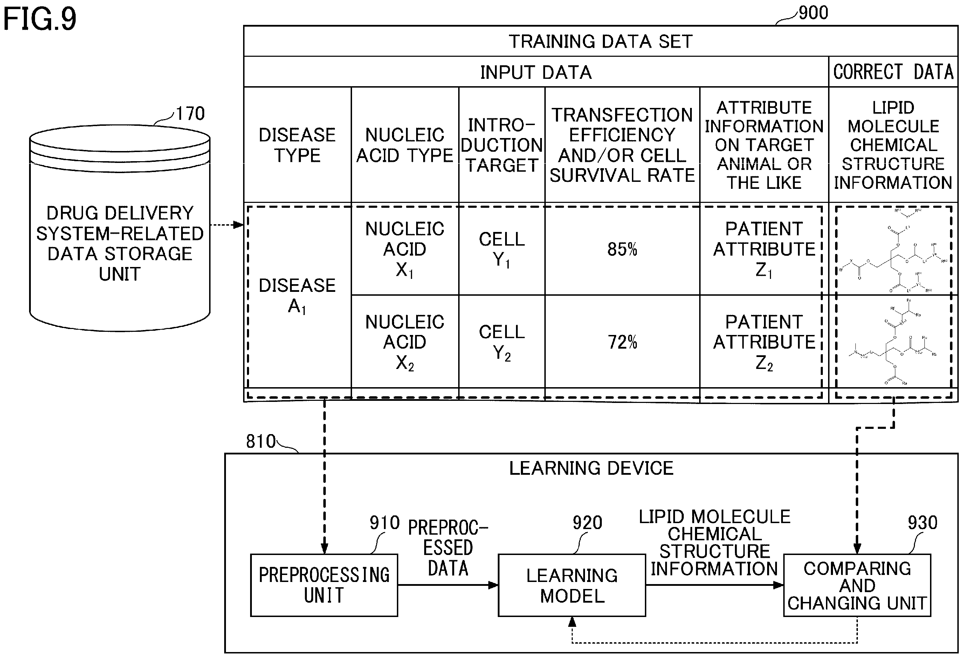

Next, a functional configuration of the learning device 810 will be described in detail. FIG. 9 is a diagram illustrating an example of the functional configuration of the learning device according to the second embodiment. In FIG. 9, a training data set 900 is an example of the training data set generated based on various data stored in the drug delivery system-related data storage unit 170. Information items of the training data (input data and correct data) are exemplified below, but are not limited thereto, and a part or all of them can be appropriately used as the training data.

As illustrated in FIG. 9, the training data set 900 includes the input data and the correct data, and in the input data, as the information items, the "disease type", the "nucleic acid type", the "introduction target", the "transfection efficiency and/or cell survival rate", and the "attribute information on the target animal or the like" are included.

In the "disease type", for example, when the target animal or the like 160 is a patient, the "disease A₁", which is a type of the disease thereof, is stored.

In the "nucleic acid type", for example, as types of the nucleic acids corresponding to the "disease A₁", a "nucleic acid X₁", a "nucleic acid X₂", and the like are stored.

In the "introduction target", for each of the "disease type" and the "nucleic acid type", information indicating a target cell, into which the particle of the complex is to be introduced, is stored. The example of FIG. 9 indicates that when the diseases type = the "disease A₁" and the nucleic acid type = the "nucleic acid X₁", the particle of the complex is introduced into the "cell Y₁", and that when the disease type = the "diseased A₁" and the nucleic acid type = the "nucleic acid X₂", the particle of the complex is introduced into the "cell Y₂".

Examples of the information indicating the target cell here include an organ or tissue in which the origin or the cell is present in a living organism, the type of the cell (e.g., a nerve cell, a parenchymal cell, a stromal cell, or the like), and the like, but are not limited thereto.

In the "transfection efficiency and/or cell survival rate", the transfection efficiency of the nucleic acid encapsulated in the particle containing the lipid molecules into a cell and/or the cell survival rate are stored. Specifically, the transfection efficiency and/or the cell survival rate, calculated from the measurement result obtained by applying the particle of a corresponding complex to a corresponding target animal or the like 160 or the cell and/or tissue 160 outside the living organism (in vitro, in situ, or ex vivo) and measuring the applied target animal or the like or the sample separated therefrom, are stored.

These training data sets may be data obtained from various public information (for example, patent publications and papers) or databases.

The example of FIG. 9 indicates that the transfection rate of the nucleic acid type = the "nucleic acid X₁" encapsulated in the particle containing the lipid molecules into the cell is "85%". Additionally, the example of FIG. 8 indicates that the transfection efficiency of the nucleic acid type = the "nucleic acid X₂" encapsulated in the particle containing the lipid molecule into the cell is "72%".

In the "attribute information on the target animal or the like", attribute information on the target animal or the like 160 (for example, the target patient) is stored. The example of FIG. 9 indicates that the particle of the complex, formed by the nucleic acid type = the "nucleic acid X₁" being encapsulated in the particle containing the lipid molecule, is introduced into the "cell Y₁" of the target patient having the attribute information on the target animal or the like = the "patient attribute Z₁". Additionally, the example of FIG. 9 indicates that the particle of the complex, formed by the nucleic acid type = the "nucleic acid X₂" being encapsulated in the particle containing the lipid molecules, is introduced into the "cell Y₂" of the target patient having the attribute information on the target animal or the like = the "patient attribute Z₂".

With respect to the above, in the correct data, as the information item, "lipid molecule chemical structure information" is included. In the "lipid molecule chemical structure information", information indicating a chemical structure of a more appropriate lipid molecule designed or selected in the past by the designer 110 based on the information included in the design preconditions 101, such as the "disease type", the "nucleic acid type", and the "introduction target" is stored.

With respect to the above, a learning program is installed in the learning device 810, and by executing the learning program, the learning device 810 functions as a preprocessing unit 910, a learning model 920, and a comparing and changing unit 930.

The preprocessing unit 910 acquires the "input data" of the training data set 900 and performs various preprocessing to generate preprocessed data suitable for input into the learning model 920. The various preprocessing performed by the preprocessing unit 910 include processing for normalizing the input data, processing for vectorizing the input data, and the like.

The learning model 920 is a model that associates the input data with the correct data (the lipid molecule chemical structure information). Specifically, the learning model 920 receives the preprocessed data notified by the preprocessing unit 910 as an input and outputs the lipid molecule chemical structure information.

Here, a learning process of updating the model parameters by backpropagating the error calculated by the comparing and changing unit 930 is performed on the learning model 920. This generates a learned model. That is, the learned model is generated by updating the model parameters of the learning model 920 so that the output of the learning model 920 approaches the correct data (the lipid molecule chemical structure information).

The comparing and changing unit 930 calculates the error by comparing the lipid molecule chemical structure information output from the learning model 920 with the correct data (the lipid molecule chemical structure information) of the training data set 900. Additionally, the comparing and changing unit 930 updates the model parameters of the learning model 920 by backpropagating the calculated error.

### <Functional configuration of the inference device>

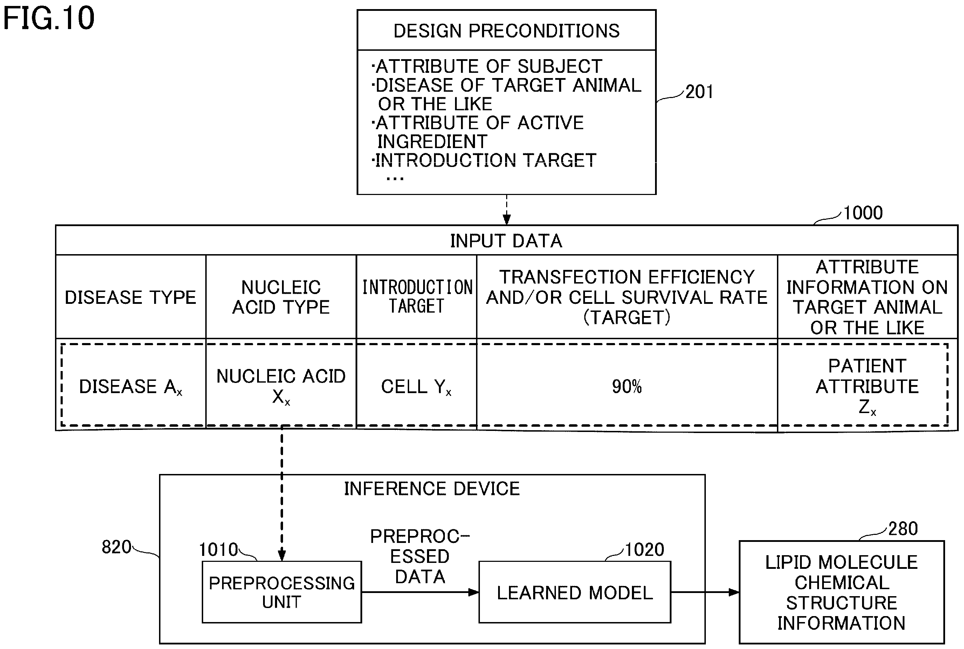

Next, a functional configuration of the inference device 820 will be described in detail. FIG. 10 is a diagram illustrating an example of the functional configuration of the inference device according to the second embodiment. As illustrated in FIG. 10, in input data 1000, information included in the design preconditions 201 (the disease type, the nucleic acid type, the introduction target, the attribute information on the target animal or the like, and the like) and a target transfection efficiency and/or a target cell survival rate are included. The input data may be data obtained from various public information (for example, patent publications and papers) or databases.

The input data 1000 is input to the inference device 820. An inference program is installed in the inference device 820, and by executing the inference program, the inference device 820 functions as a preprocessing unit 1010 and a learned model 1020.

The preprocessing unit 1010 is another example of an acquiring unit, and has the same function as that of the preprocessing unit 910 included in the learning device 810. Specifically, the preprocessing unit 1010 acquires the input data 1000 and performs various preprocessing on the acquired input data 1000 to generate preprocessed data.

The learned model 1020 is a learned model generated by the learning device 810 performing the learning process, and infers the lipid molecule chemical structure information 280 by using the preprocessed data notified by the preprocessing unit 1010 as an input.

As described above, according to the inference device 820, the lipid molecule chemical structure information can be directly inferred from the information included in the design preconditions. This achieves the design or selection of a chemical structure of a more appropriate lipid molecule forming the particle encapsulating the nucleic acid 240 without depending on the experience or know-how of the designer 110. That is, according to the inference device 820 in the second embodiment, the operation of designing or selecting the chemical structure of the lipid molecule forming the particle encapsulating the nucleic acid can be supported.

### <Flow of a process of inferring the lipid molecule chemical structure information>

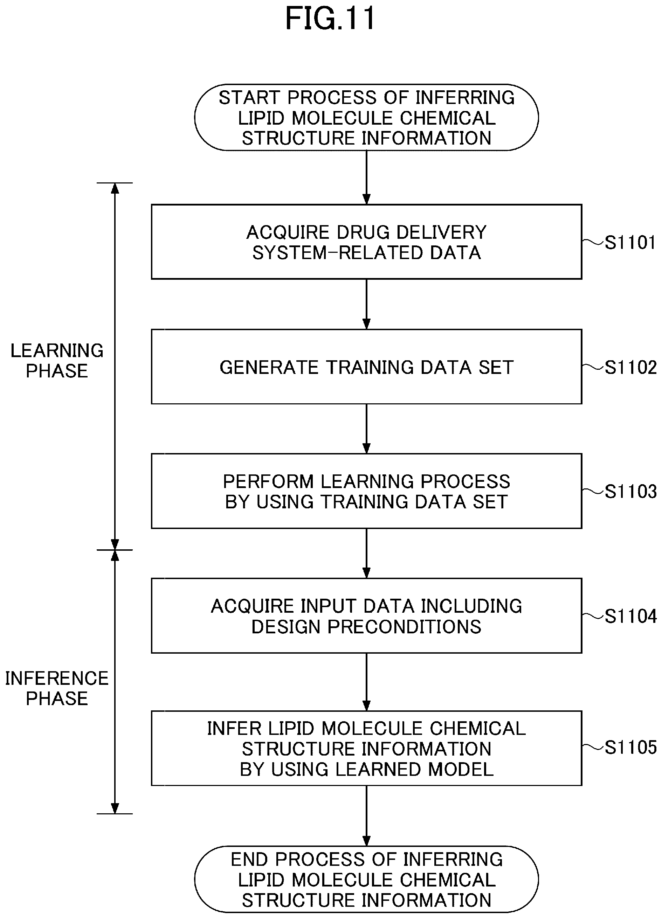

Next, a flow of a process of inferring the lipid molecule chemical structure information performed by the drug delivery system examination process 800 will be described. FIG. 11 is an example of a flowchart illustrating the flow of a process of inferring the lipid molecule chemical structure information.

In step S1101, the learning device 810 acquires various data from the drug delivery system-related data storage unit 170.

In step S1102, the learning device 810 generates the training data set 900 by using the acquired various data sets.

In step S1103, the learning device 810 performs the learning process on the learning model 920 by using the training data set 900 to generate the learned model 1020.

In step S1104, the inference device 820 acquires the input data (for example, the input data 1000) including information included in the design preconditions 201, and the target transfection efficiency and/or the target cell survival rate.

In step S1105, the inference device 820 executes the learned model 1020 by inputting the acquired input data (for example, the input data 1000) into the learned model 1020, to infer the lipid molecule chemical structure information.

### <Summary>

As is apparent from the above description, the inference device 820 according to the second embodiment acquires the input data including the preconditions for designing or selecting the lipid molecule forming the particle encapsulating the nucleic acid, and includes the learned model generated by performing the learning process on the learning model that associates the input data including the precondition for designing or selecting the lipid molecule forming the particle encapsulating the nucleic acid with the lipid molecule chemical structure information. The learned model infers the lipid molecule chemical structure information associated with the newly acquired input data.

With this, according to the inference device 820 in the second embodiment, the design or selection of a chemical structure of a more appropriate lipid molecule forming the particle encapsulating the nucleic acid can be achieved without depending on the experience or know-how of the designer. That is, according to the inference device 820 in the second embodiment, the operation of designing or selecting the chemical structure of the lipid molecule forming the particle encapsulating the nucleic acid can be supported.

### [Third embodiment]

In the second embodiment, the following case has been described.
- Generating the training data set based on various data accumulated in the drug delivery system-related data storage unit 170
- Generating the learned model by performing the learning process by using the generated training data set
- Inferring the lipid molecule chemical structure information by inputting the input data including the design precondition into the generated learned model
- Forming the particle of the complex by encapsulating the nucleic acid with the particle containing the lipid molecule generated based on the inferred chemical structure information
- Applying the formed particle of the complex to the target animal or the like, or the cell and/or tissue outside the living organism (in vitro, in situ, or ex vivo), or the like

With respect to the above, in the third embodiment, the following case will be described.
- Acquiring the design preconditions from a user
- Inferring the lipid molecule chemical structure information by inputting input data including the acquired design precondition into the generated learned model
- Providing the inferred chemical structure information to the user
- Forming, by the user, the particle of the complex by the user generating the lipid molecule based on the provided chemical structure information, and encapsulating the nucleic acid with the particle containing the generated lipid molecule
- Collecting data related to the drug delivery system from the user, obtained by applying the formed particle of the complex to the target animal or the like, or the cell and/or tissue outside the living organism (in vitro, in situ or ex vivo), or the like
- Updating the learned model by updating the training data set by using the collected data relating to the drug delivery system and performing the learning process again

With this, according to the third embodiment, a more appropriate lipid molecule forming a particle encapsulating each of various types of nucleic acids possessed by the user can be searched, and a great deal of know-how for searching for a more appropriate lipid molecule forming the particle encapsulating the nucleic acid can be accumulated. In the following, the third embodiment will be described focusing on differences from the first and second embodiments.

### <Application example of the inference service providing system>

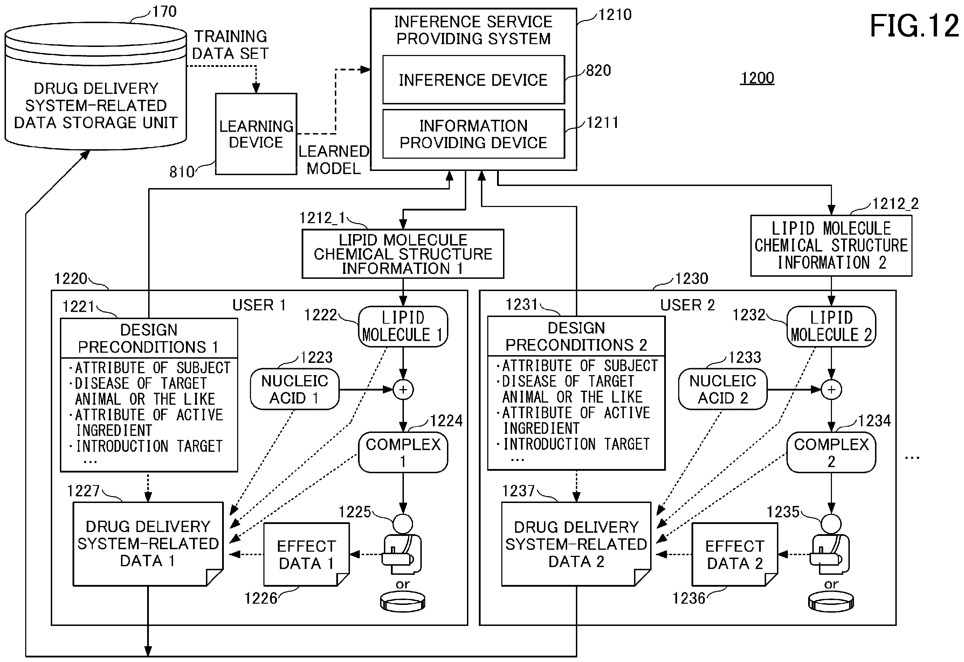

First, an application example of the inference service providing system according to the third embodiment to the drug delivery system examination process, which provides the lipid molecule chemical structure information to a user, will be described. FIG. 12 is a diagram illustrating the application example of the inference service providing system according to the third embodiment.

Specifically, FIG. 12 indicates a case in which in response to a request from a user 1220 (user name = "user 1"), a user 1230 (user name = "user 2"), ..., and the like, an inference service providing system 1210 provides the lipid molecule chemical structure information to each user.

As illustrated in FIG. 12, the inference service providing system 1210 includes the inference device 820 and an information providing device 1211.

Among these, the inference device 820 is the same as the inference device 820 described in the above-described second embodiment with reference to FIG. 8 and FIG. 10. Specifically, the inference device 820 executes the learned model generated by the learning device 810 to infer the lipid molecule chemical structure information from the input data including the design precondition.

With respect to the above, the information providing device 1211 functions as an acquiring unit. Specifically, the information providing device 1211 acquires design preconditions 1221 (information name = "design preconditions 1") and design preconditions 1231 (information name = "design preconditions 2") from the user 1220 and the user 1230, respectively.

Additionally, the information providing device 1211 generates input data respectively including the acquired design preconditions 1221 and the acquired design preconditions 1231, and notifies the inference device 820 of the input data. This causes the inference device 820 to execute the learned model to infer the lipid molecule chemical structure information as in the second embodiment.

Additionally, the information providing device 1211 functions as a providing unit. Specifically, in response to the notification of the input data including the design preconditions 1221, the information providing device 1211 provides the user 1220 with lipid molecule chemical structure information 1212_1 (information name = "lipid molecule chemical structure information 1") inferred by the inference device 820. Additionally, in response to the notification of the input data including the design precondition 1231, the information providing device 1211 provides the user 1230 with lipid molecule chemical structure information 1212_2 (information name = "lipid molecule chemical structure information 2") inferred by the inference device 820.

Further, the information providing device 1211 also functions as a charging unit, and charges each user when providing the lipid molecule chemical structure information to each user. As a result, the inference service providing system 1210 can receive a payment commensurate with the inference service of the lipid molecule chemical structure information. Here, the charging refers to processing for recording an amount of money to be paid by each user to the inference service providing system 1210.

The user 1220 transmits, to the inference service providing system 1210 via a terminal, which is not illustrated, the design preconditions 1221, such as attribute information on a target animal or the like 1225, a disease type of the target animal or the like 1225, an attribute of a nucleic acid 1223, and a target into which a particle of a complex 1224 is introduced.

Additionally, the lipid molecule chemical structure information 1212_1 corresponding to the design preconditions 1221 is provided to the user 1220 from the inference service providing system 1210 via the terminal, which is not illustrated, in exchange for the payment.

Additionally, the user 1220 generates a lipid molecule 1222 (the lipid molecule type = "lipid molecule 1") based on the provided lipid molecule chemical structure information 1212_1. Additionally, the user 1220 forms the particle of the complex 1224 (the complex type = "complex 1") by encapsulating the nucleic acid 1223 (the nucleic acid type = "nucleic acid 1") with the particle containing the generated lipid molecule 1222.

Additionally, the user 1220 applies the formed particle of the complex 1224 to the target animal or the like 1225, or the cell and/or tissue 1225 outside the living organism (in vitro, in situ, or ex vivo), or the like. A change caused by introducing the particle of the complex 1224 into the specific cell of the target animal or the like 1225 (or a change caused by the introduction into the specific cell 1225 outside the living organism (in vitro, in situ, or ex vivo)) is measured by various measurement methods and measurement devices and is output as effect data 1226 (the data name = "effect data 1").

In the effect data 1226, the transfection efficiency of the nucleic acid 1223 encapsulated by the particle containing the lipid molecule 1222 into the cell and/or the cell survival rate calculated from the measurement result are included.

Further, the user 1220 registers various data acquired in a sequence flow of the drug delivery system examination process 1200 in the drug delivery system-related data storage unit 170. As illustrated in FIG. 12, in drug delivery system-related data 1227 (data name = "drug delivery system-related data 1") registered in the drug delivery system-related data storage unit 170, the following and the like are included.
- "Design preconditions 1"
- "Lipid molecule chemical structure information 1"
- Chemical structure information on "nucleic acid 1"
- Chemical structure information on "complex 1"
- "Effect data 1"

The drug delivery system-related data 1227 registered by the user 1220 may be data obtained by the user 1220 from various public information (for example, patent publications and papers) or databases.

Here, in response to the user 1220 having registered the drug delivery system-related data 1227, the inference service providing system 1210 returns, to the user 1220, a part of the payment to be received for providing the lipid molecule chemical structure information 1212_1. That is, in the inference service providing system 1210, the details of the charge applied to the user 1220 is changed.

Similarly, the user 1230 transmits, to the inference service providing system 1210 via the terminal, which is not illustrated, the design preconditions 1231, such as attribute information on a target animal or the like 1235, the disease type when the target animal or the like is a patient, an attribute of a nucleic acid 1233 (for example, a type and chemical structure information about the nucleic acid 1233), and a target into which a particle of a complex 1234 is introduced.

Additionally, the lipid molecule chemical structure information 1212_2 corresponding to the design preconditions 1231 is provided to the user 1230 from the inference service providing system 1210 via the terminal, which is not illustrated, in exchange for the payment.

Additionally, the user 1230 generates the lipid molecule 1232 (the lipid molecule type = "lipid molecule 2") based on the provided lipid molecule chemical structure information 1212_2. Additionally, the user 1230 forms the particle of the complex 1234 (the complex type = "complex 2") by encapsulating the nucleic acid 1233 (the nucleic acid type = "nucleic acid 2") with the particle containing the generated lipid molecule 1232.

Additionally, the user 1230 applies the formed particle of the complex 1234 to the target animal or the like 1235, the cell and/or tissue 1235 outside the living organism (in vitro, in situ, or ex vivo), or the like. A change caused by introducing the particle of the complex 1234 into a specific cell in the living organism (in vivo) of the target animal or the like 1235 (or a change caused by the introduction into the specific cell 1235 outside the living organism (in vitro, in situ, or ex vivo)) is measured by various measuring methods and measuring devices, and is output as effect data 1236 (the data name = "effect data 2").

In the effect data 1236, the transfection efficiency of the nucleic acid 1233 encapsulated by the particle containing the lipid molecule 1232 into the cell and/or the cell survival rate calculated from the measurement result are included.

Further, the user 1230 registers various data acquired in a sequence flow of the drug delivery system examination process 1200 in the drug delivery system-related data storage unit 170. As illustrated in FIG. 12, drug delivery system-related data 1237 (data name = "drug delivery system-related data 2") registered in the drug delivery system-related data storage unit 170 includes the following and the like.
- "Design Precondition 2"
- "Lipid molecule chemical structure information 2"
- Chemical structure information on "nucleic acid 2"
- Chemical structure information on "complex 2"
- "Effect data 2"

Here, in response to the user 1230 having registered the drug delivery system-related data 1237, the inference service providing system 1210 can return a part of the payment to be received for providing the lipid molecule chemical structure information 1212_2 to the user 1230. That is, the inference service providing system 1210 can change the details of the charge applied to the user 1230.

As described above, by the inference service providing system 1210 providing the lipid molecule chemical structure information in response to a request from each user, a more appropriate lipid molecule forming the particle encapsulating each of various types of nucleic acids can be searched.

Additionally, in the drug delivery system-related data storage unit 170, drug delivery system-related data for various types of nucleic acids can be accumulated. Additionally, in the data stored in the drug delivery system-related data storage unit 170, data available from various public information (for example, patent publications and papers) and databases may be included. Further, in the learning device 810, by updating the training data set by using newly accumulated drug delivery system-related data and performing the learning process again, the learned model can be updated.

As a result, according to the inference service providing system 1210 in the third embodiment, a great deal of know-how for searching for a more appropriate lipid molecule forming the particle encapsulating the nucleic acid can be accumulated. That is, according to the inference service providing system 1210 in the third embodiment, the operation of designing or selecting the chemical structure of the lipid molecule forming the particle encapsulating the nucleic acid can be supported.

### <Flow of an inference service providing process>

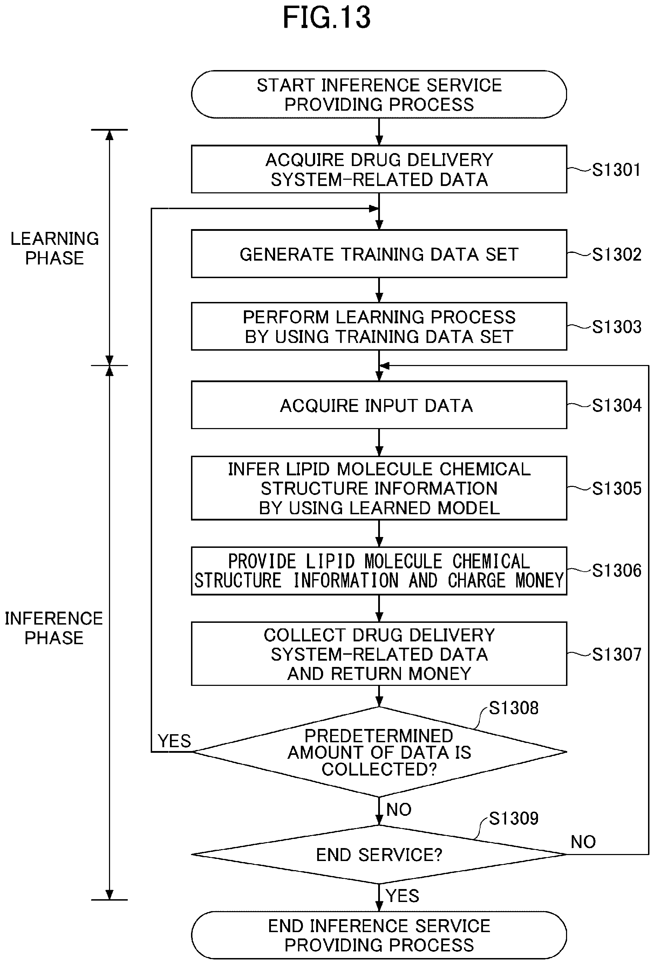

Next, a flow of an inference service providing process of the drug delivery system examination process 1200 will be described. FIG. 13 is an example of a flowchart illustrating the flow of the inference service providing process.

In step S1301, the learning device 810 acquires various data from the drug delivery system-related data storage unit 170.

In step S1302, the learning device 810 generates the training data set 900 by using the acquired various data.

In step S1303, the learning device 810 performs the learning process on the learning model 920 by using the training data set 900 to generate the learned model 1020.

In step S1304, the inference device 820 of the inference service providing system 1210 acquires the input data generated by the information providing device 1211 based on the design preconditions transmitted by the user.

In step S1305, the inference device 820 of the inference service providing system 1210 executes the learned model 1020 by inputting the acquired input data into the learned model 1020 to infer the lipid molecule chemical structure information.

In step S1306, the information providing device 1211 of the inference service providing system 1210 provides the lipid molecule chemical structure information inferred by the inference device 820 to the user who has transmitted the design preconditions, and charges the user.

In step S1307, when the data related to the drug delivery system has been collected from the user in response to the information providing device 1211 having provided the lipid molecule chemical structure information to the user, the inference service providing system 1210 can return a part of the payment to the user.

In step S1308, the inference service providing system 1210 determines whether a predetermined amount of the data related to the drug delivery system has been collected from the user.

When it is determined in step S1308 that the predetermined amount of data has been collected (YES in step S1308), the process returns to step S1302. In this case, the training data set is generated based on the newly registered predetermined amount of the data, and the learning process is performed again.

When it is determined in step S1308 that the predetermined amount of the data has not been collected (NO in step S1308), the process proceeds to step S1309.

In step S1309, the information providing device 1211 of the inference service providing system 1210 determines whether to end the inference service providing process. When it is determined in step 1309 to continue the inference service providing process (NO in step S1309), the process returns to step S1304.

When it is determined in step S1309 to end the inference service providing process (YES in step S1309), the inference service providing process is ended.

### <Summary>

As is apparent from the above description, the inference service providing system 1210 according to the third embodiment acquires the preconditions for designing or selecting the lipid molecule forming the particle encapsulating the nucleic acid from the user, and includes the learned model generated by performing the learning process on the learning model that associates the input data including a precondition for designing or selecting the lipid molecule forming the particle encapsulating the nucleic acid with the lipid molecule chemical structure information. The lipid molecule chemical structure information inferred by the learned model by inputting the input data including the preconditions acquired from the user is provided to the user who has transmitted the preconditions.

With this, according to the inference service providing system 1110 in the third embodiment, a more appropriate lipid molecule forming the particle encapsulating each of various types of nucleic acids presented by the user can be searched.

Additionally, the inference service providing system 1210 according to the third embodiment collects the data related to the drug delivery system from the user in response to having provided the lipid molecule chemical structure information.

With this, according to the inference service providing system 1210 in the third embodiment, a great deal of know-how for searching for a more appropriate lipid molecule forming the particle encapsulating the nucleic acid can be accumulated. That is, according to the inference service providing system 1210 in the third embodiment, the operation of designing or selecting the chemical structure of the lipid molecule forming the particle encapsulating the nucleic acid can be supported.

### [Fourth embodiment]

In the third embodiment described above, it is described that the data related to the drug delivery system is collected from the user in response to having provided the lipid molecule chemical structure information, and the learning process is performed again when the predetermined amount of data is accumulated.

With respect to the above, in the fourth embodiment, in response to having provided the lipid molecule chemical structure information, the transfection efficiency and/or the cell survival rate are acquired from the user. Additionally, in the fourth embodiment, a reinforcement learning process is performed on a reinforcement learning model by using a reward calculated based on the acquired transfection efficiency and/or cell survival rate. As a result, according to the fourth embodiment, the inference accuracy can be improved with the inference service being provided, and a great deal of know-how for searching for a more appropriate lipid molecule forming the particle encapsulating the nucleic acid can be accumulated.

In the following, the fourth embodiment will be described focusing on differences from the third embodiment.

### <Application example of the inference service providing system>

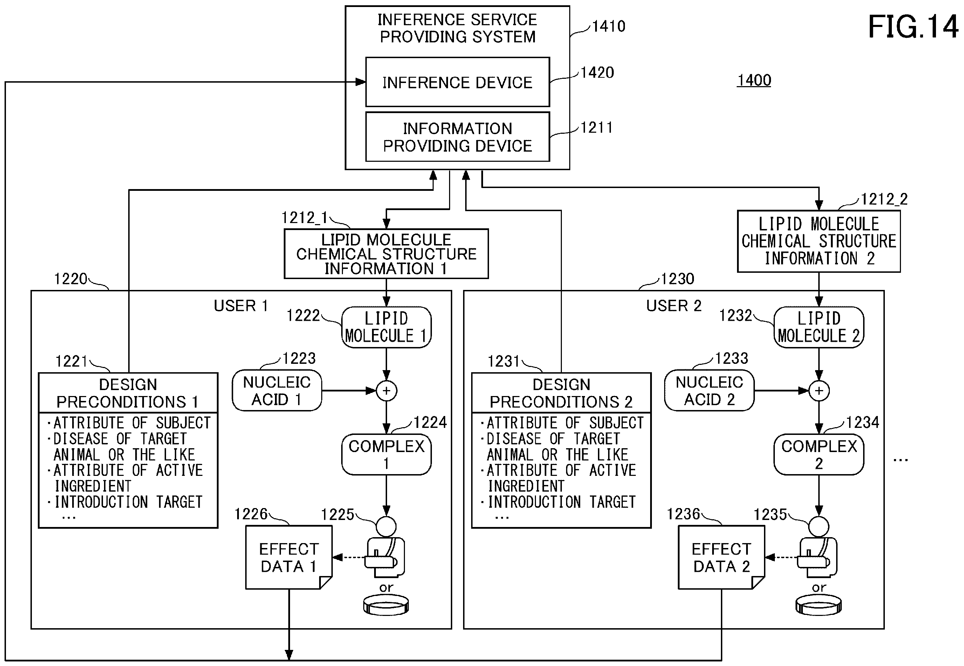

First, an application example of an inference service providing system according to the fourth embodiment to the drug delivery system examination process, which provides the lipid molecule chemical structure information to the user, will be described. FIG. 14 is a diagram illustrating the application example of the inference service providing system according to the fourth embodiment.

Specifically, FIG. 14 illustrates, similarly with FIG. 12, a case in which an inference service providing system 1410 provides the lipid molecule chemical structure information to each user in response to a request from the user 1220, the user 1230, and the like. Here, as illustrated in FIG. 14, in a drug delivery system examination process 1400, the inference service providing system 1410 includes an inference device 1420 and the information providing device 1211.

Among these, the information providing device 1211 has the same function as that of the information providing device 1211 described in the above third embodiment with reference to FIG. 12. Specifically, when the design preconditions are transmitted by each user, the information providing device 1211 generates respective input data and notifies the inference device 1420 of the input data. Additionally, in response to having notified the input data, the information providing device 1211 acquires the lipid molecule chemical structure information inferred by the inference device 1420 and provides the acquired information to a corresponding user.

Further, as in the third embodiment, the information providing device 1211 charges each user for providing the lipid molecule chemical structure information. This enables the inference service providing system 1410 to receive a payment commensurate with the inference service of the lipid molecule chemical structure information.

With respect to the above, in response to the chemical structure information on the lipid molecule being provided to the user by the information providing device 1211, the inference device 1420 acquires the effect data from the user. Additionally, the inference device 1420 calculates the reward based on the transfection efficiency and/or the cell survival rate included in the acquired effect data, and updates the model parameters of the reinforcement learning model based on the calculated reward.

Additionally, the inference device 1420 executes the reinforcement learning model by inputting the input data newly notified by the information providing device 1211 into the reinforcement learning model, the model parameters of which have been updated, and newly infers the lipid molecule chemical structure information.

Here, the processes performed by the user 1220 and the user 1230 in FIG. 14 are substantially the same as the process described with reference to FIG. 12 in the third embodiment described above, and thus description thereof is omitted here. It should be noted that the user 1220 transmits the effect data 1226 to the inference service providing system 1410. At this time, the effect data 1226 to be transmitted includes the transfection efficiency of the nucleic acid 1223 encapsulated in the particle containing the lipid molecule 1222 into the cell and/or the cell survival rate.

Similarly, the user 1230 transmits the effect data 1236 to the inference service providing system 1410. At this time, the effect data 1236 to be transmitted includes the transfection efficiency of the nucleic acid 1233 encapsulated in the particle containing the lipid molecule 1232 into the cell and/or the cell survival rate.

The data transmitted to the inference service providing system may be data available from various public information (for example, patent publications and papers) or databases.

Here, in response to the user 1220 having transmitted the effect data 1226, the inference service providing system 1410 can return a part of the payment for providing the lipid molecule chemical structure information 1212_1 to the user 1220. That is, the inference service providing system 1410 can change the details of the charge applied to the user 1220.

Similarly, in response to the user 1230 having transmitted the effect data 1236, the inference service providing system 1410 can return a part of the payment for providing the lipid molecule chemical structure information 1212_2 to the user 1230. That is, the inference service providing system 1410 can change the details of the charge applied to the user 1230.

As described above, every time the inference service providing system 1410 provides the lipid molecule chemical structure information in response to a request from each user, the inference service providing system 1410 acquires the effect data from each user to perform the reinforcement learning process on the reinforcement learning model.

With this, according to the inference service providing system 1410 in the fourth embodiment, the reinforcement learning process can be performed on the reinforcement learning model for many types of nucleic acids, and the inference accuracy can be improved with the inference service being provided.

As a result, according to the inference service providing system 1410 in the fourth embodiment, a great deal of know-how for searching for a more appropriate lipid molecule forming the particle encapsulating the nucleic acid can be accumulated. That is, according to the inference service providing system 1410 in the fourth embodiment, the operation of designing or selecting a chemical structure of a more appropriate lipid molecule forming the particle encapsulating the nucleic acid can be supported.

### <Functional configuration of the inference device>

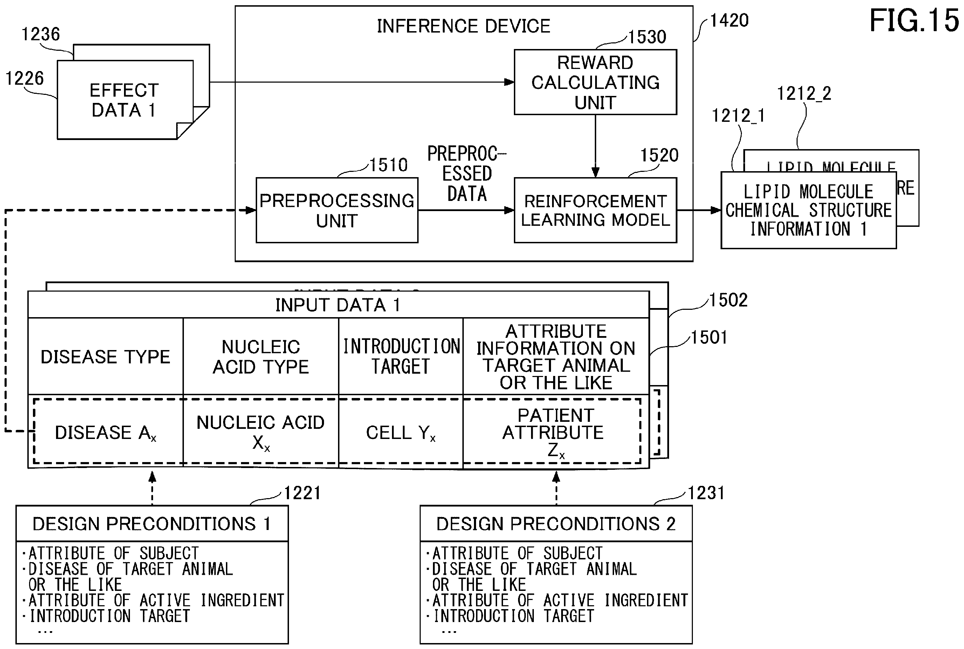

Next, a functional configuration of the inference device 1420 will be described in detail. FIG. 15 is a diagram illustrating an example of the functional configuration of the inference device. An inference program is installed in the inference device 1420, and by executing the inference program, the inference device 1420 functions as a preprocessing unit 1510, a reinforcement learning model 1520, and a reward calculating unit 1530.

The preprocessing unit 1510 has the same function as that of the preprocessing unit 910 included in the learning device 810, and generates preprocessed data by performing various types of preprocessing on the input data 1501, 1502, and the like. Here, in the input data 1501, the design preconditions 1221 transmitted by the user 1220 are included. Additionally, in the input data 1502, the design preconditions 1231 transmitted by the user 1230 are included.

The reinforcement learning model 1520 receives the preprocessed data notified by the preprocessing unit 1510 as an input, and infers the lipid molecule chemical structure information 1212_1, 1212_2, and the like.

The reward calculating unit 1530 functions as a calculating unit, and calculates rewards based on the transfection efficiencies and/or the cell survival rates included in the effect data 1226, 1236 and the like transmitted by the users 1220 and 1230. Here, the reward calculating unit 1530 calculates the reward such that the reward is maximized by the transfection efficiency and/or the cell survival rate being increased.

Additionally, the reward calculating unit 1530 performs the reinforcement learning processing for updating the model parameters of the reinforcement learning model 1520 based on the calculated reward.

### <Flow of the inference service providing process>

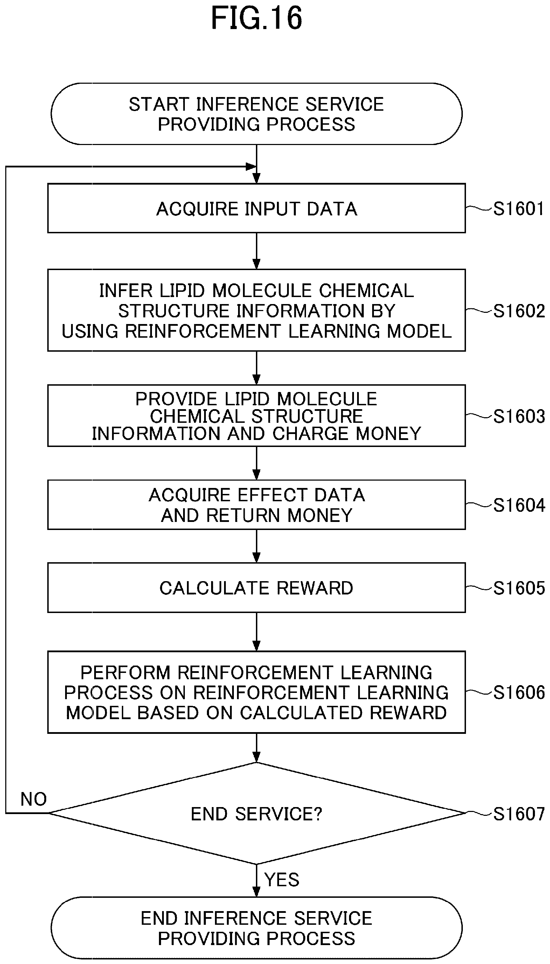

Next, a flow of the inference service providing process of the drug delivery system examination process 1400 will be described. FIG. 16 is another example of the flowchart illustrating the flow of the inference service providing process.

In step S1601, the inference device 1420 of the inference service providing system 1410 acquires the input data generated by the information providing device 1211 based on the design preconditions transmitted by the user.

In step S1602, the inference device 1420 of the inference service providing system 1410 executes the reinforcement learning model 1520 by inputting the acquired input data into the reinforcement learning model 1520, and infers the lipid molecule chemical structure information.

In step S1603, the information providing device 1211 of the inference service providing system 1410 provides the lipid molecule chemical structure information inferred by the inference device 1420 to the user who has transmitted the design preconditions, and charges the user.

In step S1604, the inference device 1420 of the inference service providing system 1410 acquires the effect data from the user in response to the information providing device 1211 having provided the lipid molecule chemical structure information to the user. Additionally, the inference device 1420 of the inference service providing system 1410 returns a part of the payment to the user who has transmitted the effect data.

In step S1605, the inference device 1420 of the inference service providing system 1410 calculates the reward based on the transfection rate and/or the cell survival rate included in the acquired effect data.

In step S1606, the inference device 1420 of the inference service providing system 1410 performs the reinforcement learning process for updating the model parameters on the reinforcement learning model based on the calculated reward.

In step S1607, the information providing device 1211 of the inference service providing system 1410 determines whether to end the inference service providing process. When it is determined in step 1607 to continue the inference service providing process (NO in step S1607), the process returns to step S1601.

When it is determined in step S1607 that the inference service providing process is to be ended (YES in step S1607), the inference service providing process is ended.

### <Summary>

As is apparent from the above description, the inference service providing system 1410 according to the fourth embodiment acquires the preconditions for designing or selecting the lipid molecule forming the particle encapsulating the nucleic acid from the user, includes the reinforcement learning model that infers the lipid molecule chemical structure information by inputting the input data including the acquired preconditions, acquires the transfection efficiency of the nucleic acid encapsulated in the particle containing the lipid molecule generated based on the chemical structure information inferred by the reinforcement learning model into the cell and/or the cell survival rate, calculates the reward, and performs the reinforcement learning process on the reinforcement learning model based on the calculated reward.

With this, according to the inference service providing system 1410 in the fourth embodiment, the inference accuracy can be improved with the inference service being provided. As a result, according to the inference service providing system 1410 in the fourth embodiment, a great deal of know-how for searching for a more appropriate lipid molecule forming the particle encapsulating the nucleic acid can be accumulated. That is, according to the inference service providing system 1410 in the fourth embodiment, the operation of designing or selecting a chemical structure of a more appropriate lipid molecule forming the particle encapsulating the nucleic acid can be supported.

### [Fifth embodiment]

In the first embodiment described above, the case is in which the inference device 220 is configured to generate the evaluation data 221 by applying the learned model 520 generated by the learning device 210 to the inference device 220 has been described.

However, the method of applying the learned model 520 is not limited to this. For example, the inference device may be configured to search for chemical structure information on a lipid molecule that satisfies a target transfection efficiency and/or a target cell survival rate. In the following, the fifth embodiment will be described focusing on differences from the first embodiment.

### <Functional configuration of the inference device>

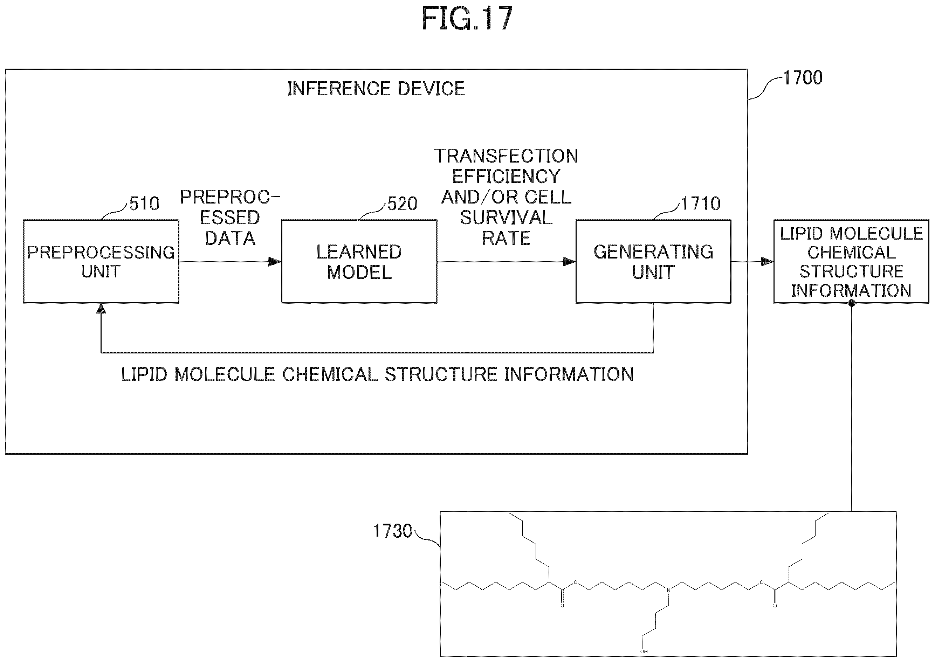

First, a functional configuration of an inference device according to the fifth embodiment will be described in detail. FIG. 17 is a diagram illustrating an example of the functional configuration of the inference device according to the fifth embodiment. As illustrated in FIG. 17, the inference device 1700 functions as the preprocessing unit 510, the learned model 520, and a generating unit 1710.

Among these, because the preprocessing unit 510 and the learned model 520 have already been described with reference to FIG. 5 in the first embodiment described above, the description thereof is omitted here.

The generating unit 1710 has a Thompson Sampling-based reinforcement learning function, for example. Specifically, the generating unit 1710 determines whether a predetermined termination condition is satisfied (for example, whether the transfection efficiency and/or the cell survival rate inferred by the learned model 520 satisfies the target transfection efficiency and/or the target cell survival rate).

When the generating unit 1710 determines that the predetermined termination condition is not satisfied, the generating unit 1710 generates the lipid molecule chemical structure information based on the transfection efficiency and/or the cell survival rate inferred by the learned model 520. Additionally, the generating unit 1710 notifies the preprocessing unit 510 of the generated lipid molecule chemical structure information.

When the generating unit 1710 determines that the predetermined termination condition is satisfied, the generating unit 1710 outputs the previously generated lipid molecule chemical structure information as the lipid molecule chemical structure information that satisfies the target transfection efficiency and/or the target cell survival rate.

In FIG. 17, the reference numeral 1730 denotes an example of the lipid molecule chemical structure information output by the generating unit 1710. The example of FIG. 17 illustrates a state in which a specific lipid molecule is generated as the lipid molecule chemical structure information that satisfies the target transfection efficiency and/or the target cell survival rate.

### <Flow of a generation process performed by the inference device>

Next, a flow of a generation process performed by the inference device 1700 for generating lipid molecule chemical structure information that satisfies the target transfection efficiency and/or the target cell survival rate will be described.

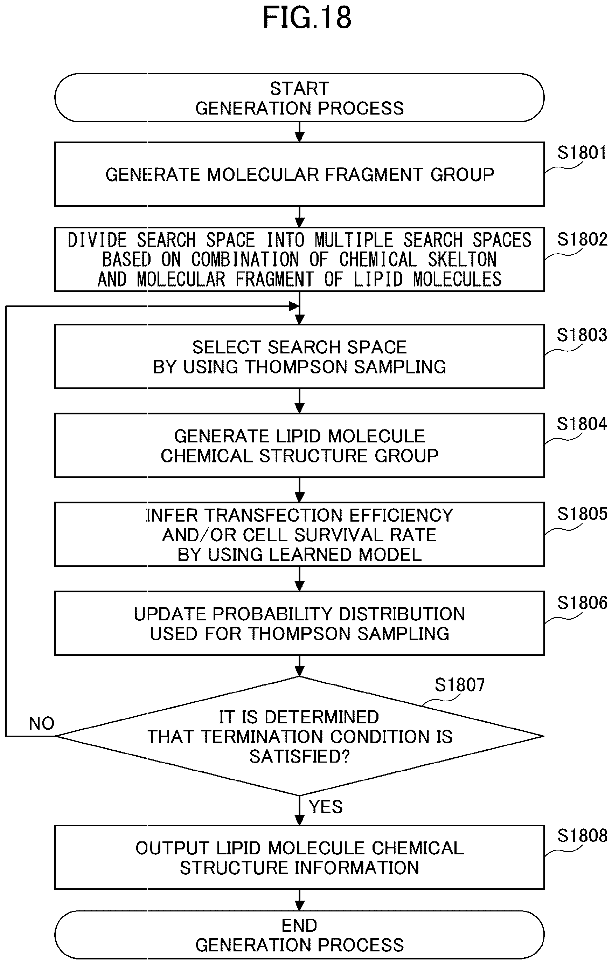

FIG. 18 is an example of a flowchart illustrating the flow of the generation process. Here, it is assumed that when the generation process illustrated in FIG. 18 is started by the inference device 1700, the target transfection efficiency and/or the target cell survival rate are set in advance in the inference device 1700.

In step S1801, the generating unit 1710 generates a molecular fragment group from chemically formable hydrocarbons in which the maximum value of the length, the degree of saturation, and the number of branches are set.

In step S1802, the generating unit 1710 combines the generated molecular fragment with the chemical skeleton group of the lipid selected by the designer 110 to generate a lipid molecule chemical structure group. At this time, the generating unit 1710 divides the generated lipid molecule chemical structure group into multiple search spaces as many as the number defined by the designer 110 in accordance with a combination of the length, the degree of saturation, the number of branches, the type of chemical skeleton, and the like of the molecular fragment.

In step S1803, the generating unit 1710 selects one search space from among the multiple search spaces divided in step S1802 by using Thompson Sampling. Here, the selecting of the one search space is nothing else other than selecting the characteristics of the search space (the combination of the length, the degree of saturation, the number of branches, the type of chemical skeleton, and the like of the molecular fragment of the lipid molecule).

In step S1804, the generating unit 1710 generates the lipid molecule chemical structure group by using the combination of the length, the degree of saturation, the number of branches, the type of chemical skeleton, and the like of the selected molecular fragment of the lipid molecule. Here, the generating unit 1710 randomly acquires multiple molecular fragments from a search space other than the selected search space with a certain probability, and generates the lipid molecule chemical structure group together with the selected molecular fragment.

In step S1805, the generating unit 1710 notifies the preprocessing unit 510 of each piece of the chemical structure information on the generated lipid molecule chemical structure group. Additionally, the preprocessing unit 510 generates a preprocessed data group suitable for input into the learned model 520 by performing various types of preprocessing on each lipid molecule chemical structure information notified by the generating unit 1710, and inputs the preprocessed data group into the learned model 520. This causes the learned model 520 to infer the transfection efficiencies and/or cell survival rates for the lipid molecule chemical structure group generated by the generating unit 1710 in step S1804.

In step S1806, the generating unit 1710 updates the probability distribution used for Thompson Sampling by using the following.
- A maximum value (an inference result) among multiple transfection efficiencies and/or cell survival rates inferred by the learned model 520
- The search space selected in step S1803

In step S1807, the generating unit 1710 determines whether a predetermined termination condition is satisfied. When it is determined in step S1807 that the predetermined termination condition is not satisfied (NO in step S1807), the process returns to step S1803.

When it is determined in step S1807 that the predetermined termination condition is satisfied (YES in step S1807), the process proceeds to step S1808.

In step S1808, the generating unit 1710 outputs the previously generated lipid molecule chemical structure information (the lipid molecule chemical structure information for which the maximum value has been inferred) as the lipid molecule chemical structure information that satisfies the target transfection efficiency and/or the target cell survival rate.

### <Summary>

As is apparent from the above description, the inference device 1700 according to the fifth embodiment includes the learned model obtained by performing the learning process on the learning model that associates the input data including at least the lipid molecule chemical structure information with the transfection efficiency of the active ingredient encapsulated in the particle containing the lipid molecule into the cell and/or the cell survival rate, includes the generating unit configured to generate, when the transfection efficiency and/or the cell survival rate associated with the input data including the newly generated lipid molecule chemical structure information is inferred by the learned model, the next new lipid molecule chemical structure information based on the inference result. The generating unit repeats the generation process of generating a next new lipid molecule chemical structure based on the inference result until a predetermined termination condition is satisfied.

With this, according to the inference device 1700 in the fifth embodiment, for example, the lipid molecule chemical structure information that satisfies the target transfection efficiency and/or the target cell survival rate can be generated. That is, according to the inference device 1700 in the fifth embodiment, the operation of designing or selecting the chemical structure information on the lipid molecule forming the particle encapsulating the nucleic acid can be supported.

### [Sixth embodiment]

In the fifth embodiment described above, the lipid molecule chemical structure information is generated in accordance with the transfection efficiency and/or the cell survival rate.

With respect to the above, in the sixth embodiment, as in the second embodiment, in addition to the transfection efficiency and/or the cell survival rate, the information included in the design preconditions 201 (the disease type, the nucleic acid type, the introduction target, the attribute information on the target animal or the like, and the like) is input. In the sixth embodiment, when the lipid molecule chemical structure information is generated, the lipid molecule chemical structure information in accordance with the information included in the design preconditions 201 (the disease type, the nucleic acid type, the introduction target, the attribute information on the target animal or the like, and the like) is generated. In the following, the sixth embodiment will be described focusing on differences from the fifth embodiment.

### <Functional configuration of the inference device>

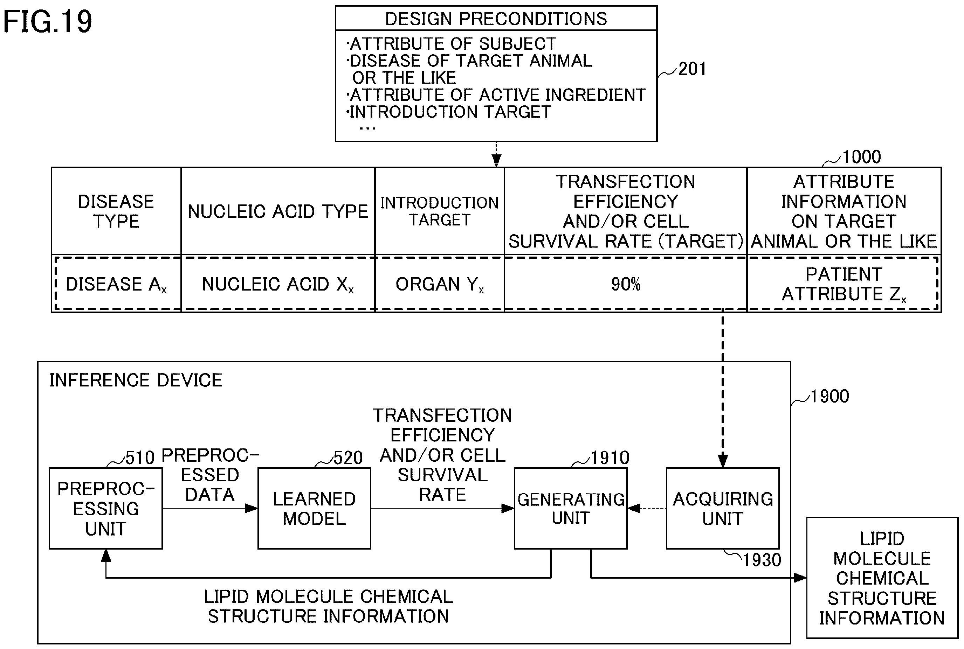

FIG. 19 is a diagram illustrating an example of a functional configuration of an inference device according to the sixth embodiment. As illustrated in FIG. 19, an inference device 1900 functions as the preprocessing unit 510, the learned model 520, a generating unit 1910, and an acquiring unit 1930.

Because the preprocessing unit 510 and the learned model 520 among these have already been described with reference to FIG. 5 in the first embodiment, the description thereof is omitted here.

The generating unit 1910 has a Thompson Sampling-based reinforcement learning function, similarly with the generating unit 1710 in FIG. 17. Specifically, the generating unit 1910 acquires the transfection efficiency and/or the cell survival rate output from the learned model 520. Additionally, the generating unit 1910 determines whether a predetermined termination condition is satisfied (for example, whether the acquired transfection efficiency and/or cell survival rate satisfy the target transfection efficiency and/or the target cell survival rate). Additionally, when the generating unit 1910 determines that the predetermined termination condition is not satisfied, the generating unit 1910 generates the lipid molecule chemical structure information based on the acquired transfection efficiency and/or cell survival rate, and notifies the preprocessing unit 510 of the generated lipid molecule chemical structure information.

Here, when generating the lipid molecule chemical structure information, the generating unit 1910 acquires the information included in the design preconditions 201 (the disease type, the nucleic acid type, the introduction target, the attribute information on the target animal or the like, and the like) notified by the acquiring unit 1930 as a predetermined constraint condition. Then, when generating the lipid molecule chemical structure information, the generating unit 1910 generates the chemical structure information by using the acquired information as the predetermined constraint condition.

When the generating unit 1910 determines that the predetermined termination condition is satisfied, the generating unit 1910 outputs the previously generated lipid molecule chemical structure information as the lipid molecule chemical structure information that satisfies the target transfection efficiency and/or the target cell survival rate.

The acquiring unit 1930 acquires the input data 1000. Additionally, the acquiring unit 1930 notifies the generating unit 1910 of the acquired input data 1000.

### <Summary>

As is apparent from the above description, the inference device 1900 according to the sixth embodiment includes the learned model obtained by performing the learning process on the learning model that associates the input data including at least the lipid molecule chemical structure information with the transfection efficiency of the active ingredient encapsulated in the particle containing the lipid molecule into the cell and/or the cell survival rate, uses the learned model to infer the transfection efficiency and/or the cell survival rate associated with the input data including the newly generated lipid molecule chemical structure information, and generates the next new lipid molecule chemical structure information based on the inferred transfection efficiency and/or cell survival rate. At this time, the next new lipid molecule chemical structure information is generated by using the information included in the design preconditions as the predetermined constraint condition. The generation process of generating the next new lipid molecule chemical structure information based on the inferred transfection efficiency and/or cell survival rate and the information included in the design preconditions is repeated until the predetermined termination condition is satisfied.

With this, according to the inference device 1900 in the sixth embodiment, the lipid molecule chemical structure information that satisfies the target transfection efficiency and/or the target cell survival rate can be generated under the predetermined constraint condition. That is, according to the inference device 1900 in the sixth embodiment, the operation of designing or selecting the chemical structure of the lipid molecule forming the particle encapsulating the nucleic acid can be supported.

### [Other Embodiments]

In the first to third embodiments, a case in which the learning device and the inference device are configured as separate devices has been described. However, the learning device and the inference device may be configured as an integrated device.

Additionally, in the third embodiment described above, a case in which the inference device 820 and the information providing device 1211 are configured as separate devices in the inference service providing system 1210 has been described. However, the inference device 820 and the information providing device 1211 may be configured as an integrated device. In this case, in the inference service providing system 1210, for example, the function of the inference device 820 and the function of the information providing device 1211 may be achieved by executing the inference service providing program in the integrated device.

Similarly, in the fourth embodiment described above, a case in which the inference device 1420 and the information providing device 1211 are configured as separate devices in the inference service providing system 1410 has been described. However, the inference device 1420 and the information providing device 1211 may be configured as an integrated device. In this case, in the inference service providing system 1410, for example, the function of the inference device 1420 and the function of the information providing device 1211 may be achieved by executing the inference service providing program in the integrated device.

Additionally, in the third and fourth embodiments described above, a case in which the information providing device generates the input data has been described. However, it may be configured such that the input data is generated by the inference device, for example.

Additionally, in the third and fourth embodiments described above, a case in which the information providing device functions as the acquiring unit, the providing unit, and the charging unit has been described. However, a part of the functions achieved by the information providing device may be achieved in the terminal of the user (or on the cloud).

Additionally, in the third and fourth embodiments described above, the details of the method of providing, by the information providing device, the lipid molecule chemical structure information to the user are not described, but the providing method performed by the information providing device may be suitably selected. For example, the information providing device may be configured to directly transmit the lipid molecule chemical structure information to the user, or may be configured to store the lipid molecule chemical structure information in a storage location accessible by the user inputting a password or the like.

Additionally, in the fifth and sixth embodiments described above, the generating unit determines whether the transfection efficiency and/or the cell survival rate inferred by the learned model 520 satisfy the target transfection efficiency and/or the target cell survival rate as the predetermined termination condition. However, the predetermined termination condition is not limited thereto, and for example, the generating unit may determine whether the generated lipid molecule chemical structure information has been updated. In this case, the generating unit determines that the predetermined termination condition is satisfied when the generating unit determines that the generated lipid molecule chemical structure information has not been updated.

Additionally, in the embodiments described above, the information items in FIG. 5, FIG. 10, FIG. 15, and the like are exemplified as the information items of the input data, but the information items of the input data are not limited thereto.

Here, the present invention is not limited to the configurations described herein, such as the configurations described in the above embodiments, combinations of the configurations with other elements, and the like. These points can be changed within a range not departing from the subject matter of the present invention, and can be appropriately determined according to the application form.

This application is based upon and claims the priority to Japanese Patent Application No. 2020-146402 filed on August 31, 2020, the entire contents of which are incorporated herein by reference.

### Description of the Reference Numerals

- 100:: drug delivery system examination process
- 170:: drug delivery system-related data storage
- 200:: drug delivery system examination process
- 210:: learning device
- 220:: inference device
- 400:: training data set
- 520:: learned model
- 800:: drug delivery system examination process
- 810:: learning device
- 820:: inference device
- 900:: training data set
- 1000:: input data
- 1020:: learned model
- 1200:: drug delivery system examination process
- 1211:: information providing device
- 1400:: drug delivery system examination process
- 1410:: inference service providing system
- 1420:: inference device
- 1520:: reinforcement learning model
- 1530:: reward calculating unit
- 1700:: inference device
- 1710:: generating unit
- 1900:: inference device
- 1910:: generating unit
- 1930:: acquiring unit

## Claims

1. An inference device comprising:
an acquiring unit configured to acquire input data including at least chemical structure information on a lipid molecule; and
a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
wherein the learned model infers a transfection efficiency and/or a cell survival rate associated with the input data newly acquired by the acquiring unit.

2. The inference device as claimed in claim 1, wherein the transfection efficiency and/or the cell survival rate used when the learning process is performed are calculated based on a measurement result measured by introducing, into the cell, the active ingredient encapsulated in the particle containing the lipid molecule having the chemical structure information that is used when the learning process is performed.

3. The inference device as claimed in claim 2, wherein the learned model is generated by updating model parameters of the learning model so that an output, obtained when the input data including at least the chemical structure information on the lipid molecule is input into the learning model, approaches the transfection efficiency and/or the cell survival rate calculated based on the measurement result.

4. The inference device as claimed in claim 1,
wherein the acquiring unit performs predetermined preprocessing on the newly acquired input data, and
wherein the learned model infers the transfection efficiency and/or the cell survival rate associated with the preprocessed input data.

5. An inference method comprising:
an acquisition step of acquiring input data including at least chemical structure information on a lipid molecule; and
an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
wherein the execution step infers, by executing the learned model, a transfection efficiency and/or a cell survival rate associated with the input data newly acquired in the acquisition step.

6. An inference program for causing a computer to perform:
an acquisition step of acquiring input data including at least chemical structure information on a lipid molecule; and
an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate,
wherein the execution step infers, by executing the learned model, a transfection efficiency and/or a cell survival rate associated with the input data newly acquired in the acquisition step.

7. A model generation method of generating a learned model by performing a learning process on a learning model that associates input data including at least chemical structure information on a lipid molecule with a transfection efficiency of an active ingredient encapsulated in a particle containing the lipid molecule into a cell and/or a cell survival rate.

8. An inference device comprising:
an acquiring unit configured to acquire input data including a precondition for designing or selecting a lipid molecule forming a particle encapsulating an active ingredient; and
a learned model generated by performing a learning process on a learning model that associates input data including a precondition for designing or selecting a lipid molecule forming a particle encapsulating an active ingredient with chemical structure information on the lipid molecule,
wherein the learned model infers chemical structure information on a lipid molecule associated with the input data newly acquired by the acquiring unit.

9. The inference device as claimed in claim 8, wherein the input data used when the learning process is performed includes a transfection efficiency of the active ingredient encapsulated in the particle containing the lipid molecule into a cell and/or a cell survival rate, calculated based on a measurement result measured by introducing, into the cell, the active ingredient encapsulated in the particle containing the lipid molecule that is designed or selected.

10. The inference device as claimed in claim 8, wherein the learned model is generated by updating model parameters of the learning model so that an output, obtained when the input data including the precondition is input into the learning model, approaches the chemical structure information on the lipid molecule used when the learning process is performed.

11. The inference device as claimed in claim 8,
wherein the acquiring unit performs predetermined preprocessing on the newly acquired input data, and
wherein the learned model infers the chemical structure information on the lipid molecule associated with the preprocessed input data.

12. An inference method comprising:
an acquisition step of acquiring input data including a precondition for designing or selecting a lipid molecule forming a particle encapsulating an active ingredient; and
an execution step of executing a learned model generated by performing a learning process on a learning model that associates input data including a precondition for designing or selecting a lipid molecule forming a particle encapsulating an active ingredient with chemical structure information on the lipid molecule,
wherein the execution step infers, by executing the learned model, chemical structure information on a lipid molecule associated with the input data newly acquired in the acquisition step.

13. An inference program for causing a computer to perform:
an acquisition step of acquiring input data including a precondition for designing or selecting a lipid molecule forming a particle encapsulating an active ingredient; and
an execution step of executing a learned model generated by performing a learning process on a learning model that associates input data including a precondition for designing or selecting a lipid molecule forming a particle encapsulating an active ingredient with chemical structure information on the lipid molecule,
wherein the execution step infers, by executing the learned model, chemical structure information on a lipid molecule associated with the input data newly acquired in the acquisition step.

14. A model generation method of generating a learned model by performing a learning process on a learning model that associates input data including a precondition for designing or selecting a lipid molecule forming a particle encapsulating an active ingredient with chemical structure information on the lipid molecule.

15. An inference service providing system comprising:
an acquiring unit configured to acquire, from a user, a precondition for designing or selecting a lipid molecule forming a particle encapsulating an active ingredient;
a learned model generated by performing a learning process on a learning model that associates input data including a precondition for designing or selecting a lipid molecule forming a particle encapsulating an active ingredient with chemical structure information on the lipid molecule; and
a providing unit configured to provide, to the user, chemical structure information on a lipid molecule inferred by the learned model by input data, including the precondition newly acquired by the acquiring unit from the user, being input.

16. The inference service providing system as claimed in claim 15, further comprising a charging unit configured to charge the user when the learned model infers the chemical structure information on the lipid molecule by the input data, including the precondition newly acquired by the acquiring unit from the user, being input.

17. The inference service providing system as claimed in claim 16, wherein the charging unit changes details of the charge applied to the user, when a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the learned model, are acquired by the user.

18. An inference service providing method comprising:
an acquisition step of acquiring, from a user, a precondition for designing or selecting a lipid molecule forming a particle encapsulating an active ingredient;
an execution step of executing a learned model generated by performing a learning process on a learning model that associates input data including a precondition for designing or selecting a lipid molecule forming a particle encapsulating an active ingredient with chemical structure information on the lipid molecule; and
a provision step of providing, to the user, chemical structure information on a lipid molecule inferred by the learned model by input data, including the precondition newly acquired in the acquisition step from the user, being input.

19. An inference program for causing a computer to perform:
an acquisition step of acquiring, from a user, a precondition for designing or selecting a lipid molecule forming a particle encapsulating an active ingredient;
an execution step of executing a learned model generated by performing a learning process on a learning model that associates input data including a precondition for designing or selecting a lipid molecule forming a particle encapsulating an active ingredient with chemical structure information on the lipid molecule; and
a provision step of providing, to the user, chemical structure information on a lipid molecule inferred by the learned model by input data including the precondition newly acquired in the acquisition step from the user being input.

20. An inference device comprising:
an acquiring unit configured to acquire input data including a precondition for designing or selecting a lipid molecule forming a particle encapsulating an active ingredient;
a reinforcement learning model configured to infer, by the input data including the precondition acquired by the acquiring unit being input, chemical structure information on the lipid molecule; and
a calculating unit configured to calculate a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated by the calculating unit.

21. The inference device as claimed in claim 20, wherein the calculating unit calculates the reward such that the reward is maximized by the transfection efficiency and/or the cell survival rate being increased.

22. The inference device as claimed in claim 20,
wherein the acquiring unit performs predetermined preprocessing on the input data, and
wherein the reinforcement learning model infers the chemical structure information on the lipid molecule by the preprocessed input data being input.

23. An inference method comprising:
an acquisition step of acquiring input data including a precondition for designing or selecting a lipid molecule forming a particle encapsulating an active ingredient;
an execution step of executing a reinforcement learning model configured to infer, by the input data including the precondition acquired in the acquisition step being input, chemical structure information on the lipid molecule; and
a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated in the calculation step.

24. An inference program for causing a computer to perform:
an acquisition step of acquiring input data including a precondition for designing or selecting a lipid molecule forming a particle encapsulating an active ingredient;
an execution step of executing a reinforcement learning model configured to infer, by the input data including the precondition acquired in the acquisition step being input, chemical structure information on the lipid molecule; and
a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated in the calculation step.

25. An inference service providing system comprising:
an acquiring unit configured to acquire, from a user, a precondition for designing or selecting a lipid molecule forming a particle encapsulating an active ingredient;
a reinforcement learning model configured to infer, by input data including the precondition acquired by the acquiring unit from the user being input, chemical structure information on the lipid molecule;
a providing unit configured to provide, to the user, the chemical structure information on the lipid molecule inferred by the reinforcement learning model; and
a calculating unit configured to calculate a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated by the calculating unit.

26. The inference service providing system as claimed in claim 25, further comprising a charging unit configured to charge the user when the providing unit provides, to the user, the chemical structure information on the lipid molecule that the reinforcement learning model infers.

27. The inference service providing system as claimed in claim 26, wherein the charging unit changes details of the charge applied to the user when the transfection efficiency of the active ingredient encapsulated in the particle containing the lipid molecule into the cell and/or the cell survival rate, calculated based on the measurement result measured by introducing, into the cell, the active ingredient encapsulated in the particle containing the lipid molecule having the chemical structure information inferred by the reinforcement learned model, are acquired by the user.

28. An inference service providing method comprising:
an acquisition step of acquiring, from a user, a precondition for designing or selecting a lipid molecule forming a particle encapsulating an active ingredient;
an execution step of executing a reinforcement learning model configured to infer, by input data including the precondition acquired in the acquisition step from the user being input, chemical structure information on the lipid molecule;
a provision step of providing, to the user, the chemical structure information on the lipid molecule inferred by the reinforcement learning model; and
a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated by the calculation step.

29. An inference service providing program for causing a computer to perform:
an acquisition step of acquiring, from a user, a precondition for designing or selecting a lipid molecule forming a particle encapsulating an active ingredient;
an execution step of executing a reinforcement learning model configured to infer, by input data including the precondition acquired in the acquisition step from the user being input, chemical structure information on the lipid molecule;
a provision step of providing, to the user, the chemical structure information on the lipid molecule inferred by the reinforcement learning model; and
a calculation step of calculating a reward based on a transfection efficiency of an active ingredient encapsulated in a particle containing the lipid molecule into a cell and/or a cell survival rate, calculated based on a measurement result measured by introducing, into the cell, the active ingredient encapsulated in the particle containing the lipid molecule having the chemical structure information inferred by the reinforcement learning model,
wherein the reinforcement learning model performs a learning process based on the reward calculated by the calculation step.

30. An inference device comprising:
a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
a generating unit configured to repeat, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.

31. The inference device as claimed in claim 30, wherein the generating unit generates the next new chemical structure information on the lipid molecule by selecting a search space from among a plurality of search spaces corresponding to combinations of a molecular fragment of formable hydrocarbons and a chemical structure of a lipid molecule, based on the inference result, and using a characteristic of the selected search space.

32. The inference device as claimed in claim 31, wherein the plurality of search spaces are different from each other in terms of a combination of a length of the molecular fragment, a degree of saturation, a number of branches, and a type of a chemical skeleton of the lipid molecule.

33. The inference device as claimed in claim 31, wherein the generating unit generates the next new chemical structure information on the lipid molecule under a predetermined constraint condition.

34. The inference device as claimed in claim 30, further comprising an acquiring unit configured to acquire a precondition for designing or selecting a lipid molecule forming a particle encapsulating an active ingredient,
wherein the generating unit generates the next new chemical structure information on the lipid molecule by using the acquired precondition as a constraint condition.

35. An inference method comprising:
an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
a generation step of repeating, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.

36. An inference program for causing a computer to perform:
an execution step of executing a learned model generated by performing a learning process on a learning model that associates input data including at least chemical structure information on a lipid molecule with a transfection efficiency of an active ingredient encapsulated in a particle containing the lipid molecule into a cell and/or a cell survival rate; and
a generation step of repeating, when a transfection efficiency and/or a cell survival rate associated with input data including new chemical structure information on the lipid molecule is inferred by the learned model, a generation process of generating next new chemical structure information on the lipid molecule based on an inference result, until a predetermined termination condition is satisfied.
